# FASCICULE DE BREVET EUROPEEN

(11) **EP 3 468 583 B1**
(45) Date de publication et mention de la délivrance du brevet: **07.07.2021**
(21) Numéro de dépôt: 17731822.7
(22) Date de dépôt: 02.06.2017
(51) Int. Cl.: A61K 38/43, A61K 38/46, A61K 38/47, A61K 45/06, A61K 31/496, A61K 31/407, A61K 31/7036, A61L 27/54, A61L 29/16, A61L 31/16, A61P 31/04, A61K 31/4709

(54) **COMPOSITION COMPRENANT AU MOINS UNE ENZYME ET AU MOINS UNE MOLÉCULE MICROBICIDE POUR LA PRÉVENTION OU LE TRAITEMENT DES INFECTIONS POST-IMPLANTATOIRES**
ZUSAMMENSETZUNG, DIE MINDESTENS EIN ENZYM UND MINDESTENS EIN MIKROBIZIDMOLEKÜL ENTHÄLT, UND VERWENDUNG ZUR VORBEUGUNG ODER BEHANDLUNG EINER POSTIMPLANTATIONSINFEKTION
COMPOSITION COMPRISING AT LEAST ONE ENZYME AND AT LEAST ONE MICROBICIDAL MOLECULE FOR TREATMENT OR PREVENTION OF POST-IMPLANTATION INFECTIONS

(30) Priorité: 08.06.2016 BE 201605427; 08.06.2016 EP 16173468
(43) Date de publication de la demande: 17.04.2019
(73) Titulaire: OneLIFE S.A., 1348 Louvain-La-Neuve (BE)
(72) Inventeur: SIALA, M. Wafi, 1150 Woluwe St. Pierre (BE); VANZIELEGHEM, M. Thomas, 1348 Louvain-la-Neuve (BE)
(74) Mandataire: Calysta NV
(86) Numéro de dépôt international: PCT/EP2017/063533
(87) Numéro de publication internationale: WO 2017/211737

(56) Documents cités:
- WO-A1-2009/121183
- US-A1- 2009 130 082
- US-A1- 2009 202 516
- G. V. TETZ ET AL: "Effect of DNase and Antibiotics on Biofilm Characteristics", ANTIMICROBIAL AGENTS AND CHEMOTHERAPY, vol. 53, no. 3, 1 mars 2009 (2009-03-01), pages 1204-1209, XP055002507, ISSN: 0066-4804, DOI: 10.1128/AAC.00471-08
- CAVALIERE ROSALIA ET AL: "The biofilm matrix destabilizers, EDTA and DNaseI, enhance the susceptibility of nontypeable Hemophilus influenzae biofilms to treatment with ampicillin and ciprofloxacin", MICROBIOLOGYOPEN, vol. 3, no. 4, août 2014 (2014-08), pages 557-567, XP002763088,
- KAPLAN JEFFREY B: "Therapeutic potential of biofilm-dispersing enzymes", INTERNATIONAL JOURNAL OF ARTIFICIAL ORGANS, WICHTIG PUBLISHING, IT, vol. 32, no. 9, 1 septembre 2009 (2009-09-01), pages 545-554, XP009165613, ISSN: 0391-3988
- JEFFREY B KAPLAN ET AL: "Extracellular DNA-dependent biofilm formation byRP62A in response to subminimal inhibitory concentrations of antibiotics", RESEARCH IN MICROBIOLOGY, ELSEVIER, AMSTERDAM, NL, vol. 162, no. 5, 25 février 2011 (2011-02-25), pages 535-541, XP028373257, ISSN: 0923-2508, DOI: 10.1016/J.RESMIC.2011.03.008 [extrait le 2011-03-12]
- BAELO AIDA ET AL: "Disassembling bacterial extracellular matrix with DNase-coated nanoparticles to enhance antibiotic delivery in biofilm infections", JOURNAL OF CONTROLLED RELEASE, vol. 209, 23 avril 2015 (2015-04-23), pages 150-158, XP029173934, ISSN: 0168-3659, DOI: 10.1016/J.JCONREL.2015.04.028
- ARCIOLA C R ET AL: "New trends in diagnosis and control strategies for implant infections", INTERNATIONAL JOURNAL OF ARTIFICIAL OR, WICHTIG PUBLISHING, IT, vol. 34, no. 9, 1 janvier 2011 (2011-01-01), pages 727-736, XP009155540, ISSN: 0391-3988, DOI: 10.5301/IJAO.2011.8784
- KAPLAN JEFFREY B ET AL: "Recombinant human DNase I decreases biofilm and increases antimicrobial susceptibility in staphylococci", JOURNAL OF ANTIBIOTICS (TOKYO), vol. 65, no. 2, février 2012 (2012-02), pages 73-77, XP002773355,
- MARTINS M ET AL: "Addition of DNase improves the in vitro activity of antifungal drugs against Candida albicans biofilms", MYCOSES, WILEY-BLACKWELL PUBLISHING LTD, GB, vol. 55, no. 1, 1 January 2012 (2012-01-01), pages 80-85, XP009155558, ISSN: 0933-7407, DOI: 10.1111/J.1439-0507.2011.02047.X [retrieved on 2011-06-12]

## Description

La présente invention se rapporte à une composition comprenant au moins une enzyme et au moins une molécule microbicide, pour utilisation dans le traitement préventif et/ou curatif d'infections du corps de mammifères, en particulier dans le traitement préventif et/ou curatif du corps humain.

Une telle composition est connue du document WO2004/062677 qui divulgue une composition comprenant obligatoirement un bactériophage et une enzyme (polysaccharide lyase) et, optionnellement, un agent antimicrobien et/ou une DNase, pour traiter les biofilms et les maladies microbiennes (infections) associées à ces biofilms. Plus particulièrement, ce document décrit une utilisation d'une telle composition dans une méthode de traitement des infections pulmonaires et gastro-intestinales liées à la présence de biofilms.

Les documents US2009/0130082 et WO2009/121183 portent sur des compositions comprenant une DNase pour disperser le biofilm et un antibiotique (agent antimicrobien) pour tuer les bactéries libérées. Selon ces documents antérieurs, ces compositions sont notamment utilisées lors de la fabrication/préparation de dispositifs médicaux pour traiter des plaies. A cette fin, les dispositifs médicaux sont coatés ou imprégnés avec une composition comprenant une DNase et un antibiotique. Plus spécifiquement, ces documents antérieurs enseignent essentiellement que de telles compositions sont utilisées pour préparer les dispositifs médicaux et pour désinfecter la peau ou le milieu environnant avant insertion ou implantation d'un dispositif médical comme par exemple un cathéter. En ce sens, les compositions divulguées dans ces documents antérieurs sont essentiellement utilisées comme solutions de rinçage pré-procédurales avant une chirurgie par exemple.

Un biofilm est une pellicule visqueuse qui se développe sur toutes les surfaces, suite à l'adhésion de microorganismes sur ces surfaces et à la sécrétion par ceux-ci de polymères qui les recouvrent et facilitent leur adhésion. Les biofilms constituent ainsi une couche de protection autour des microorganismes et représentent une source récurrente de contamination du milieu environnant qui pose des problèmes majeurs en termes de santé, par exemple dans les milieux hospitaliers.

Plus spécifiquement, l'accumulation de polymères sécrétés par les bactéries crée une matrice composée essentiellement de polysaccharides, d'ADN, de protéines ainsi que de lipides qui protège ces microorganismes des agressions extérieures et qui présente une très forte résistance aux procédures de nettoyage et de désinfection conventionnelles. Les microorganismes se développent donc aisément au sein de cette matrice protectrice et contaminent le milieu environnant en constituant un réservoir particulièrement critique et difficile à éliminer.

Il est reconnu que la problématique de la présence de biofilms est double. Premièrement, comme indiqué plus haut, ceux-ci représentent une source de contamination permanente et très difficile à éliminer par des moyens conventionnels, même les plus agressifs. En effet, les désinfectants classiques sont très souvent inefficaces car il est observé qu'ils ne parviennent pas à atteindre les microorganismes qui sont protégés par la matrice du biofilm composée de polysaccharides, d'ADN, de protéines et de lipides.

Deuxièmement, un biofilm est mixte, en ce sens qu'il est initialement développé par certaines souches bactériennes mais qu'il peut en abriter d'autres, ces souches vivant et se développant en colonies. Or, ces colonies favorisent la communication entre bactéries et, entre autre, l'échange et la propagation de gènes de résistance portés par certaines bactéries. Les biofilms résultant de ces échanges de gènes sont alors encore plus difficiles à éliminer et il faut recourir à des moyens de désinfection ou de traitement de plus en plus puissants qui se heurtent toutefois fréquemment à des problèmes de résistance et/ou de tolérance majeurs.

La matrice de protection des bactéries formant les biofilms est si résistante qu'elle constitue une véritable barrière protégeant les bactéries des agents microbicides (antibiotiques et/ou biocides) qui pourraient agir contre les microorganismes et donc contre les infections liées à la présence de biofilms, dont les infections du corps humain associées à la présence de biofilm. Actuellement, les traitements classiques à base de différents antibiotiques et/ou de différents biocides, même lorsqu'ils sont, dans certains cas, en association avec d'autres composés (détergents formulés et/ou séquestrants et/ou dispersants et/ou tensioactifs), n'agissent pas de manière suffisamment efficace car ils ne pénètrent pas ou seulement de façon très limitée le biofilm dans son épaisseur. Par ailleurs, les microbicides peuvent être inhibés par certaines molécules composant cette matrice. Par conséquent, les traitements actuels ne sont que partiellement efficaces, ceci uniquement à la surface du biofilm, la matrice du biofilm protégeant efficacement les bactéries de phénomènes de déshydratation, de l'action des antibiotiques et des biocides (et plus généralement des molécules microbicides), de la phagocytose et des acides. En ce sens, il est d'ailleurs généralement admis que les biofilms présentent une résistance jusqu'à 1000 fois supérieure aux microbicides par rapport à une bactérie planctonique (non protégée par un biofilm).

En milieu hospitalier et vétérinaire, la situation est d'autant plus critique que de nombreux microorganismes responsables de la formation de biofilms sont détectés en de nombreux endroits, tant au niveau des individus patients / animaux (plaies, système respiratoire, ...) qu'au niveau de l'environnement (salle d'opération, matériel chirurgical, équipements de maintenance de ce matériel, endoscopes, sondes urinaires, cathéters, équipement médical, appareil de dialyse ou de ventilation assistée des individus, ...) et des surfaces (sols, murs, tables d'opération, ...).

De tout ceci, il ressort que les biofilms constituent une réelle problématique, tout particulièrement dans le domaine des soins de santé (hôpitaux, cabinets dentaires...) et des soins vétérinaires. Cette problématique est d'autant plus critique que les biofilms impliquent des bactéries responsables d'infections potentiellement mortelles chez les individus, par exemple chez des individus développant une infection provoquée par les bactéries Staphylocoques ou encore les Enterobacteriaceae qui se révèlent être résistantes à des antibiotiques de dernière génération (par exemple aux imipénèmes et aux carbapénèmes). Il convient donc de prendre toutes les précautions possibles afin d'éviter la formation et le développement de biofilms, ce qui est d'autant plus important lorsque des interactions avec le corps de mammifères, en particulier avec le corps humain, c'est-à-dire des interventions d'ordre chirurgical, sont réalisées.

En ce sens, la mise en place de dispositifs médicaux dans le corps de mammifères, en particulier dans le corps humain (prothèses, implants - plaques, vis, grilles,... -, cathéters, sondes urinaires...) constitue une intervention médicale critique, ces dispositifs médicaux ayant à séjourner plusieurs jours voire plusieurs mois ou années dans le corps suite à leur mise en place. En effet, dès lors qu'il s'agit de l'implantation d'un dispositif médical dans le corps, ce dispositif constitue presque inévitablement un point d'entrée, un potentiel vecteur et une source potentielle de microorganismes (dont les bactéries) pouvant, par la suite, développer des biofilms au sein du corps de mammifères, en particulier au sein du corps humain, ceci avec tous les risques associés.

La présence de bactéries sur les dispositifs médicaux implantables, typiquement la présence des bactéries Staphylococcus aureus; Staphylococcus epidermidis; Klebsiella pneumoniae; Enterococcus faecalis; Escherichia coli et Pseudomonas aeruginosa, ou d'autres pathogènes des mammifères, peut avoir plusieurs origines dont les principales sont une contamination du dispositif médical implantable par contact avec la peau de l'individu (humain/animal) lors de son implantation, une contamination par les microorganismes présents dans l'air des salles d'opérations et des cabinets médicaux / vétérinaire ou une contamination par dispersion sanguine de bactéries au départ d'une source secondaire.

D'autres origines de la présence de microorganismes sur les dispositifs médicaux peuvent être mentionnées comme par exemple les contaminations aérosols (contaminants présents dans l'air), les contaminations aqueuses et les contaminations par contact avec une surface déjà contaminée.

Généralement, il est admis que ce sont les contaminations dites péri-opératoires (qui ont lieu dans le contexte d'une intervention médicale) qui sont à l'origine des infections se développant suite à la pose d'un dispositif médical (implantation) dans le corps de mammifères, en particulier dans le corps humain, ceci suite au développement de biofilms. Par exemple, pour les cathéters/sondes urinaires et sanguins, des contaminations péri-opératoires sont classiquement observées puisque ces dispositifs médicaux sont fortement sujets à des contaminations lors de leur utilisation, ceci notamment au vu de la région du corps où ils doivent être implantés.

L'adhésion des biofilms peut être favorisée par des dispositifs médicaux implantés eux-mêmes présentant des parties rugueuses où les forces de cisaillement sont moindres et où les surfaces d'adhésion sont plus importantes. Il semble également que des surfaces hydrophobes favorisent l'adhérence de biofilms sur les dispositifs médicaux implantés. De plus, les fluides corporels peuvent constituer un revêtement à la surface des dispositifs médicaux qui favorisent l'adhésion de certaines espèces microbiennes. Par exemple, le fibrinogène et le collagène contribuent à l'adhésion des bactéries du genre Staphylococcus.

A ce jour, afin de minimiser au mieux toute introduction de bactéries ou d'autres microorganismes (levures, moisissures et virus) au sein du corps de mammifères, en particulier au sein du corps humain, lors de la mise en place d'un dispositif médical implantable, toute une série de mesures doivent être strictement respectées. En ce sens, les opérateurs doivent faire preuve de précaution en se désinfectant les mains et en désinfectant correctement la peau de l'individu humain ou animal à l'endroit où sera effectuée l'introduction du dispositif médical implantable. Les opérateurs doivent aussi veiller à manipuler le moins possible les dispositifs médicaux implantables lors de leur mise en place, ce qui requière un certain savoir-faire. Par ailleurs, la stérilité de l'environnement de travail doit également être assurée tout comme celle des outils médicaux utilisés dans ces procédures.

D'autres alternatives sont actuellement développées, comme par exemple la mise au point de dispositifs médicaux implantables dont le design réduit les taux d'infections. Des implants avec des caractéristiques particulières sont également envisagés afin de minimiser les surfaces d'adhérence, notamment en proposant des surfaces hydrophiles, anioniques ou comprenant un revêtement (coating) spécifique.

Toutefois, malheureusement, on observe encore très fréquemment le développement d'infections post-implantatoires liées au développement de biofilm dans le corps de mammifères, en particulier dans le corps humain, ces infections étant développées suite à la mise en place d'un dispositif médical, lequel, même après avoir subi un éventuel traitement pré-implantatoire (une stérilisation ou un revêtement par exemple), est régulièrement responsable de la contamination de tissus et/ou de muqueuses internes du corps de mammifères, en particulier du corps humain. Cette contamination des tissus est d'autant plus rapide et dommageable qu'en raison du traumatisme lié à l'intervention chirurgicale de pose du dispositif médical implantable, les défenses immunitaires de l'individu humain ou animal peuvent être (localement) réduites et ainsi permettre le développement aisé d'inflammations aigües.

Très souvent, parfois en marge d'une inflammation aigüe, s'observe une inflammation dite chronique due au dispositif médical implanté n'étant pas cliniquement inerte vis-à-vis des tissus environnants et/ou aux microorganismes se trouvant dans et autour du dispositif médical implanté (c'est-à-dire au niveau du site d'implantation du dispositif médical implantable). A ce stade plus avancé du développement de l'inflammation, peut avoir lieu une déstructuration du tissu (par exemple osseux), ce qui crée un site préférentiel où les microorganismes peuvent encore mieux se localiser et se développer sous forme de biofilm, un site où les défenses immunitaires sont localement réduites et donc un site où des infections peuvent apparaître et se développer d'autant plus facilement. Notons encore que l'inflammation peut être telle qu'elle empêche une intégration correcte de l'implant au sein du corps des mammifères, en particulier au sein du corps humain.

S'en suit, dans le corps de mammifères, en particulier dans le corps humain, le développement de biofilms tant au niveau du dispositif médical implantable lui-même que dans les tissus environnants, ce qui peut conduire à des situations selon lesquelles une antibiothérapie lourde doit être mise en place. Dans des cas extrêmes, doit même être réalisée une nouvelle intervention chirurgicale ayant pour objet un retrait du dispositif médical implantable avant la mise en place d'un nouveau dispositif médical supposé être stérile. Toutefois, comme vu plus haut, une antibiothérapie (par exemple par voie veineuse) s'avère être très souvent inefficace, ne parvenant à éliminer que les symptômes mais pas la cause de l'infection, c'est-à-dire le biofilm microbien.

La procédure chirurgicale de remplacement du dispositif médical implantable n'est pas non plus une solution puisqu'elle donne lieu à une réouverture de la zone d'implantation et à un déplacement du dispositif médical implantable où s'est développé le biofilm avec le risque significatif de le disperser plus encore (cassure, fragmentation du biofilm et libération de bactéries). Dans un tel cas de figure, les bactéries protégées par le biofilm peuvent se répandre dans le corps, ce qui peut compliquer la situation clinique plutôt que de la résoudre. Pratiquement, il n'est par ailleurs pas toujours possible de retirer un implant directement suite à sa mise en place lorsqu'une infection liée à la présence de biofilm se déclare, notamment lorsque l'implant contribue à la stabilité osseuse ou assure une fonction vitale dans le corps. Notons qu'il est reconnu que les implantations secondaires d'autres dispositifs médicaux de remplacement comportent le risque d'entrainer l'infection du nouveau dispositif implanté, cette dernière étant due à la dispersion du biofilm au départ de l'implant initialement posé.

Par ailleurs, le traitement d'infections développées aux sites d'implantation de dispositifs médicaux implantables est tout à fait différent de celui mis en œuvre pour lutter contre d'autres infections liées à la présence de biofilms au niveau du corps de mammifères, en particulier au niveau du corps humain. A titre d'exemple, une infection pulmonaire qui serait due à la présence de biofilms au niveau des alvéoles pulmonaires ne sera pas traitée de la même façon qu'une infection résultant de la pose d'un dispositif médical implantable comme par exemple un pacemaker ou une prothèse de genou. En ce sens, bien que des compositions destinées à des traitements pré-implantatoires existent, leurs applications ne sont certainement pas forcément compatibles pour des traitements post-implantatoires dès lors que l'effet recherché et les contraintes rencontrées sont différentes.

Ceci résulte du fait que les microorganismes colonisant et se développant sur les dispositifs médicaux implantés sont de nature totalement différente dès lors qu'ils se développent dans des conditions optimales de température (température interne corporelle de l'ordre de 37 à 38°C) et en présence de nutriments essentiels tels que les sucres et les protéines présents dans les fluides corporels. En effet, les biofilms se développant sur et à proximité des dispositifs médicaux implantés présentent des ratios entre polymères et entre populations (colonies) bactériennes tout à fait particuliers, ce qui implique que ces biofilms se caractérisent par une morphologie et une composition spécifique leur conférant notamment une résistance accrue aux traitements classiques d'élimination par exemple à l'aide de molécules microbicides (antibiotiques et/ou biocides). Par ailleurs, chaque type d'implant a tendance à être infecté par certains types de microorganismes, ce qui est en grande partie lié à l'environnent dans lequel est localisé l'implant, ainsi qu'à la surface de l'implant lui-même. Par exemple, pour les cathéters urinaires, plus de 50% des infections sont causées par E. coli, K. pneumoniae et E. faecalis. Pour les cathéters sanguins, il s'agit plutôt majoritairement des bactéries de type Staphylocoques.

Il existe donc un réel besoin de procurer une composition d'élimination des biofilms qui puisse permettre de faire face à ces problématiques puisque les solutions actuelles de traitements pré-implantatoires, d'antibiothérapie lourde et de remplacement des dispositifs médicaux implantables par voie chirurgicale post-implantatoires ne permettent malheureusement pas d'éviter le développement, à un site d'implantation, d'infections post-implantatoires liées à la présence de biofilm dans le corps des mammifères, en particulier dans le corps humain et n'offrent pas de solution curative efficace contre ces infections post-implantatoires dans le corps des mammifères, en particulier dans le corps humain.

Pour résoudre ce problème, il est prévu suivant l'invention une composition comprenant au moins une enzyme, étant la désoxyribonucléases et au moins une molécule microbicide étant un antibiotique comme produits de combinaison, pour un emploi simultané, séparé ou échelonné dans le temps, pour utilisation dans le traitement préventif et/ou curatif d'infections à un site d'implantation, lesdites infections étant des infections post-implantatoires du corps de mammifères, en particulier des infections post-implantatoires du corps humain.

Par les termes « infections post-implantatoires du corps de mammifères, en particulier infections post-implantatoires du corps humain », on entend, au sens de la présente invention, des infections qui se développent à un site et/ou au niveau d'un site d'implantation dans le corps de mammifères, en particulier dans le corps humain (in-vivo, in-situ, en place) suite à la mise en place d'un dispositif médical implantable (implant, prothèse, sonde gastrique / urinaire, drains, cathéters, stents, ...). Ces infections se développent à un site et/ou au niveau d'un site d'implantation tant sur le dispositif médical implanté qu'à proximité de ce dernier, par exemple dans les tissus, l'os et les cartilages et/ou les muqueuses corporelles internes. Plus particulièrement, au sens de la présente invention, les infections post-implantatoires du corps de mammifères, en particulier du corps humain, sont des infections causées par le développement, après implantation et à un site d'implantation, de microorganismes présents sous forme de biofilms suite à l'implantation d'un dispositif médical prévu pour séjourner plusieurs heures, voire plusieurs jours, semaines, mois ou années dans le corps, ceci ne correspondant pas à un sondage où le dispositif médical ne fait que transiter un bref laps de temps dans le corps. Plus spécifiquement, au sens de la présente invention, les infections post-implantatoires du corps de mammifères, en particulier du corps humain, sont des infections causées par un séjour d'au moins 12 heures, plus particulièrement d'au moins 24 heures d'un dispositif médical implanté dans le corps

Par les termes « molécule microbicide », on entend, au sens de la présente invention, une molécule naturelle ou synthétique dont les propriétés lui permettent de détruire (tuer) des microorganismes ou de bloquer la croissance des microorganismes. Au sens de la présente invention, les termes « molécule microbicide » signifient les molécules antibiotiques.

Précisons encore que, au sens de la présente invention, c'est bien une infection de type post-implantatoire qui est traitée de façon préventive ou curative au site d'implantation, ceci en particulier suite à la mise en place d'un dispositif médical implantable. Il ne s'agit pas ici, comme c'est le cas par exemple pour les compositions décrites dans les documents US2009/0130082 et WO2009/121183, d'un traitement préprocédural ou d'une préparation de dispositifs médicaux implantables mais bien d'un traitement appliqué après implantation (post-implantatoire) et au site d'implantation.

Dans le cadre de la présente invention, il a été mis en évidence qu'une utilisation d'une telle composition permet de traiter efficacement, au site et/ou au niveau du site d'implantation, les infections post-implantatoires du corps de mammifères, en particulier du corps humain, liées à la présence de biofilms, ceci sans endommager de façon irréversible les tissus ou les muqueuses internes (altération, toxicité) corporelles de façon à ne pas aggraver la situation clinique du patient (humain ou animal). En outre, il a été mis en évidence que la composition selon l'invention permet de déstructurer le biofilm sous l'action d'au moins une enzyme et de tuer la majeure partie des colonies bactériennes présentes initialement au sein du biofilm au site d'implantation. L'action simultanée, séparée ou échelonnée dans le temps d'au moins une molécule microbicide et d'au moins une enzyme selon l'invention est cruciale puisqu'une libération des microorganismes peut permettre à ces derniers de se disséminer dans le corps avec tous les risques que cela peut présenter dont une délocalisation de l'infection potentiellement vers d'autres zones plus sensibles du corps de mammifères, en particulier du corps humain.

De façon inattendue, il a été mis en évidence qu'une utilisation d'une composition selon l'invention (au moins une enzyme et au moins une molécule microbicide), uniquement composée de composants pharmaceutiquement acceptables injectables ou compatibles avec le corps de mammifères, en particulier avec le corps humain, mais pouvant comprendre, sans toutefois être nécessaire, d'autres composants, permet de traiter les infections post-implantatoires au site et/ou au niveau du site d'implantation en déstructurant efficacement et rapidement les biofilms pourtant hautement résistants et particuliers développés au niveau et à proximité et/ou à la surface des dispositifs médicaux implantés.

Une telle déstructuration de ces biofilms pourtant tout à fait spécifiques permet aux molécules microbicides (antibiotiques et/ou biocides) d'agir efficacement sur les microorganismes (bactéries et/ou levures et/ou archées et/ou virus et/ou prions et/ou moisissures) responsables de ces infections. Il n'était certainement pas attendu qu'une composition, notamment utilisée pour traiter des infections pulmonaires ou pour nettoyer des outils médicaux, puisse être utilisée de façon tout aussi efficace pour lutter, à un site et/ou au niveau d'un site d'implantation, contre des infections post-implantatoires impliquant des biofilms très résistants et très spécifiques dont la morphologie et les composants (ratio de polymères, ratio des différentes colonies bactériennes) sont tout à fait atypiques de part leur association avec des dispositifs médicaux implantés dans un environnement propice au développement de biofilms.

A l'inverse des traitements par antibiothérapies lourdes et des interventions chirurgicales visant le remplacement d'un dispositif médical implanté infecté et responsable d'une infection, un traitement, à un site et/ou au niveau d'un site d'implantation des infections post-implantatoires du corps de mammifères, en particulier du corps humain, liées à la présence de biofilms, par utilisation d'une composition suivant l'invention s'est montré être hautement efficace. En ce sens, une utilisation d'une composition selon l'invention assure systématiquement un traitement de l'infection liée à la présence de biofilm au niveau du site d'implantation dès lors qu'il a été montré qu'au moins une enzyme contenue dans la composition selon l'invention est capable de déstructurer rapidement et de façon polyvalente de nombreux biofilms pourtant spécifiques, permettant ainsi assurément aux molécules microbicides (antibiotiques) d'atteindre les bactéries dépourvues de toute matrice protectrice.

Dans le cadre de la présente invention, contre toute attente, une utilisation d'une composition selon l'invention a été identifiée comme étant à même de traiter efficacement, au site et/ou au niveau du site d'implantation, des infections liées à la présence de biofilms particuliers formés par différentes associations de colonies bactériennes et présentant des caractéristiques structurelles et morphologiques très spécifiques de par leur association avec des dispositifs médicaux implantés. En particulier, il a été observé que la composition selon l'invention assure, au site et/ou au niveau du site d'implantation, une déstructuration en profondeur dans toute l'épaisseur du biofilm même si ce dernier se développe dans des conditions optimales et présente par conséquent une résistance accrue aux traitements visant à l'éliminer.

Un traitement reposant sur une utilisation d'une composition selon l'invention a été également identifié comme permettant, de façon surprenante, de réaliser un traitement ciblé efficace et rapide des infections post-implantatoires du corps de mammifères, en particulier du corps humain, liées à la présence de biofilms, c'est-à-dire un traitement in-situ à l'endroit même de développement de l'infection (au site d'implantation), ceci sans avoir à pratiquer la moindre intervention chirurgicale et sans endommager de façon irréversible les tissus et/ou les muqueuses internes corporelles (altération, déstructuration, toxicité, ...). Par exemple, dans le cas d'une sonde urinaire dont la mise en place (implantation) entraine une infection (infection urinaire) liée à la présence de biofilm, il est envisagé que la composition selon l'invention puisse être injectée dans la sonde urinaire de telle sorte à inonder cette dernière pour simultanément en éliminer le biofilm source de l'infection et traiter l'infection. Dans le cas d'une prothèse, par exemple d'une prothèse du genou, un traitement in-situ de l'infection, qui serait causée par le développement de biofilm sur et à proximité de la prothèse, pourrait être réalisé par simple infiltration ou injection (injection intra-articulaire par exemple) de la composition selon l'invention au niveau du genou.

La composition utilisée selon l'invention s'est donc montrée être à la fois polyvalente et efficace en permettant de traiter rapidement, au site d'implantation, les infections post-implantatoires liées à la présence de biofilms tout à fait particuliers pour lesquels, à ce jour, seules une antibiothérapie lourde ou une intervention chirurgicale tentent d'apporter des solutions se révélant toutefois n'être que partiellement voire pas du tout efficaces comme indiqué plus haut. En outre, une utilisation d'une composition selon l'invention a été identifiée comme permettant d'assurer une rapide intégration du dispositif médical implanté, notamment en permettant une cicatrisation plus rapide au niveau du site d'implantation, ceci grâce à l'absence de biofilm assurée par la composition selon l'invention. Enfin, de façon surprenante et avantageuse, il a été déterminé qu'une composition selon l'invention n'est pas cytotoxique alors qu'elle est pourtant hautement efficace pour détruire (tuer) les bactéries initialement protégées par les biofilms et pour éliminer les biofilms et les microorganismes des surfaces traitées.

Comme il ressortira des exemples présentés ci-après, différentes combinaisons d'au moins une enzyme étant la désoxyribonucléases avec au moins une molécule microbicide, unantibiotique permettent de traiter efficacement divers infections liées à la présence de biofilms formés par divers espèces et diverses souches bactériennes. Les compositions selon l'invention sont donc polyvalentes et permettent de traiter une large gamme d'infections post-implantatoires. Ladite au moins une enzyme de la composition suivant l'invention pour utilisation dans le traitement, à un site d'implantation, d'infections post-implantatoires du corps de mammifères, en particulier du corps humain, est choisie dans le groupe constitué des désoxyribonucléases (DNases).

De préférence, ladite au moins une enzyme de la composition suivant l'invention pour utilisation dans le traitement, à un site d'implantation, d'infections post-implantatoires du corps de mammifères, en particulier du corps humain, est présente en un volume administrable compris entre 100 et 100.000 µL.

Avantageusement, ladite au moins une enzyme de la composition suivant l'invention pour utilisation dans le traitement, à un site d'implantation, d'infections post-implantatoires du corps de mammifères, en particulier du corps humain, est présente en une concentration comprise entre 0,01 et 1000 mg/L. Dans le cas où ladite au moins une enzyme de la composition suivant l'invention est administrée sous forme d'une poudre (lyophilisat) ou d'un cachet par exemple, ladite enzyme est présente en une quantité en poids sec comprise entre 0,01 et 50.000 mg/kg par rapport au poids sec total de ladite poudre (lyophilisat) ou au poids sec total dudit cachet.

Préférentiellement, ladite au moins une molécule microbicide de la composition suivant l'invention pour utilisation dans le traitement, à un site d'implantation, d'infections post-implantatoires du corps de mammifères, en particulier du corps humain, est présente en un volume administrable compris entre 100 et 100.000 µL.

De préférence, ladite au moins une molécule microbicide de la composition suivant l'invention pour utilisation dans le traitement, à un site d'implantation, d'infections post-implantatoires du corps de mammifères, en particulier du corps humain, est présente en une concentration comprise entre 0,01 et 1000 mg/L. Dans le cas où ladite au moins une molécule microbicide de la composition suivant l'invention est administrée sous forme d'une poudre (lyophilisat) ou d'un cachet par exemple, ladite molécule microbicide est présente en une quantité en poids sec comprise entre 0,01 et 10.000 mg/kg par rapport au poids sec total de ladite poudre (lyophilisat) ou au poids sec total dudit cachet.

Ladite au moins une molécule microbicide de la composition suivant l'invention pour utilisation dans le traitement, à un site d'implantation, d'infections post-implantatoires du corps de mammifères, en particulier du corps humain, est choisie dans le groupe constitué des fluoroquinolones, des glucopeptides, des lipoglucopeptides, de l'acide fusidique, des pénicillines, des céphalosporines, des carbapenèmes, des monobactames, des polymixines, des béta-lactames, des macrolides, des lincosamides, des oxazolidinones, des phénicoles, des tétracyclines, des aminoglycosides, des rifamycines, des nitrofuranes, des sulfamides, des nitroimidazoles et de leurs mélanges.

Avantageusement, la composition selon l'invention pour utilisation dans le traitement, à un site d'implantation, d'infections post-implantatoires du corps de mammifères, en particulier du corps humain, se présente sous forme d'une solution stérile aqueuse injectable ou non, à diluer ou non dans de l'eau pour préparation injectable ou sous forme d'une poudre soluble, préférentiellement sous forme d'un lyophilisat.

De préférence, la composition selon l'invention pour utilisation dans le traitement, à un site d'implantation, d'infections post-implantatoires du corps de mammifères, en particulier du corps humain, comprend un véhicule et/ou un excipient pharmaceutiquement acceptable comme par exemple un agent stabilisant prévu pour assurer la stabilité de la composition au stockage de telle sorte que la composition ne subisse pas de dégradation avant utilisation.

De façon avantageuse, la composition selon l'invention pour utilisation dans le traitement, à un site d'implantation, d'infections post-implantatoires du corps de mammifères, en particulier du corps humain, est conditionnée dans un contenant stérile, par exemple dans un flacon stérile muni d'un opercule au travers duquel peut être injectée de l'eau pour préparation injectable de telle sorte à former la composition suivant l'invention.

Préférentiellement, lesdites infections post-implantatoires du corps de mammifères, en particulier du corps humain, traitées, à un site d'implantation, avec la composition selon l'invention sont des infections post-implantatoires de bactéries Staphylococcus aureus, Staphylococcus epidermis, Escherichia coli, Enterococcus faecalis, Klebsiella pneumoniae et Pseudomonas aeruginosa. A titre d'exemples, des infections post-implantatoires au sens de la présente invention peuvent être les infections urinaires (cathéters), les peri-implantites dentaires (implants endo-osseux, implants zygomatiques, implants orthodontiques, plaques de contention), les endocardites (valves cardiaques, stents), les plaies chirurgicales (point d'entrée de cathéters), les otites (drains), les infections pulmonaires, du larynx et de la gorge (tubes pour respirateur artificiel), les infections des sinus (drains), les infections rénales (stents, cathéters), les infections gastriques (sondes), hépatiques (stents), pancréatiques (stents), entériques (sondes) et du côlon (sondes), les infections des os et cartilages (plaques, vis et prothèses), les infections du système cérébral (stents) et les infections circulatoires (cathéters).

Selon la présente invention, les infections peuvent être également causées par des levures (par exemple par Candida albicans), par des organismes causant des mycoses (par exemple par Aspergillus) et par d'autres bactéries (par exemple par Proteus mirabilis, Enterobacter, Citrobacter ou encore Acinetobacter).

De préférence, selon l'invention, lesdites infections post-implantatoires du corps de mammifères, en particulier du corps humain, sont des infections localisées à un site d'implantation d'un dispositif médical implantable, par exemple d'une sonde, d'un cathéter ou d'une prothèse.

Avantageusement, la composition selon l'invention pour utilisation dans le traitement, à un site d'implantation, d'infections post-implantatoires du corps de mammifères, en particulier du corps humain, est prévue pour abattre d'au moins 1 Log10, de préférence d'au moins 2 Log10, préférentiellement d'au moins 5 Log10 des microorganismes responsables d'infections post-implantatoires du corps de mammifères, en particulier du corps humain.

La présente invention porte également sur une utilisation d'une composition comprenant au moins une enzyme et au moins une molécule microbicide comme produits de combinaison, pour un emploi simultané, séparé ou échelonné dans le temps, pour le traitement, à un site d'implantation, d'infections post-implantatoires du corps de mammifères, en particulier du corps humain.

De préférence, l'utilisation d'une composition selon l'invention comprenant au moins une enzyme et au moins une molécule microbicide s'effectue par injection, par infiltration, par irrigation, par ingestion ou par application percutanée. Au sens de la présente invention, ladite administration peut s'effectuer via un gel, une pommade, une crème ou via un patch. Notons que la présente invention prévoit une administration simultanée, séparée ou échelonnée dans le temps des produits de combinaison que sont ladite au moins une enzyme et ladite au moins une molécule microbicide, ceci n'imposant certainement pas que les modes (méthodes) d'administration de ces produits de combinaison soient identiques.

La présente invention porte aussi sur un procédé de traitement, à un site d'implantation, d'infections post-implantatoires du corps de mammifères, en particulier du corps humain, avec une composition comprenant au moins une enzyme et au moins une molécule microbicide comme produits de combinaison, pour un emploi simultané, séparé ou échelonné dans le temps, ledit procédé comprenant les étapes suivantes :
- administration, à un site d'implantation d'un dispositif médical implantable, d'une composition comprenant au moins une enzyme et au moins une molécule microbicide,
- déstructuration, par action de ladite au moins une enzyme de ladite composition administrée, d'un biofilm présent au niveau dudit site d'implantation dudit dispositif médical implantable, et
- destruction de bactéries et/ou blocage de la croissance de bactéries libérées au départ dudit biofilm, par action de ladite au moins une molécule microbicide de ladite composition administrée.

Avantageusement, selon le procédé de traitement, à un site d'implantation, d'infections post-implantatoires du corps de mammifères, en particulier du corps humain, ladite administration est effectuée par injection, par infiltration, par irrigation, par ingestion ou par application percutanée. Au sens de la présente invention, ladite administration peut s'effectuer via un gel, une pommade, une crème, une solution liquide ou encore via un patch.

D'autres caractéristiques, détails et avantages de l'invention ressortiront des exemples et des figures annexés.

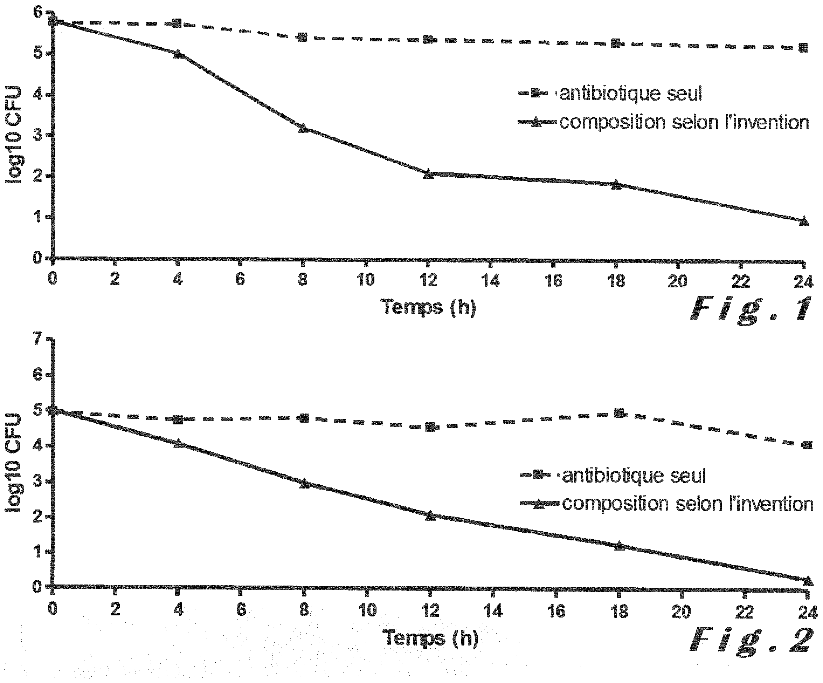

La figure 1 est un graphique qui illustre la viabilité des bactéries d'un isolat de S. aureus (isolé au départ d'un pacemaker implanté) ayant développé un biofilm, suite à une mise en contact soit avec une composition selon l'invention (la ciprofloxacine comme molécule antibiotique à 3,2 mg/L + 0,025% de DNase I, 0,01% de dispersine B et 0,05% de cellulase), soit avec une composition comprenant uniquement la ciprofloxacine comme molécule antibiotique à 3,2 mg/L.
La figure 2 est un graphique qui illustre la viabilité des bactéries d'un isolat de E. coli (isolé au départ d'un cathéter urinaire implanté) ayant développé un biofilm, suite à une mise en contact soit avec une composition selon l'invention (la ciprofloxacine comme molécule antibiotique à 3,2 mg/L + 0,025% de DNase I, 0,01% de dispersine B et 0,05% de cellulase), soit avec une composition comprenant uniquement la ciprofloxacine comme molécule antibiotique à 3,2 mg/L.

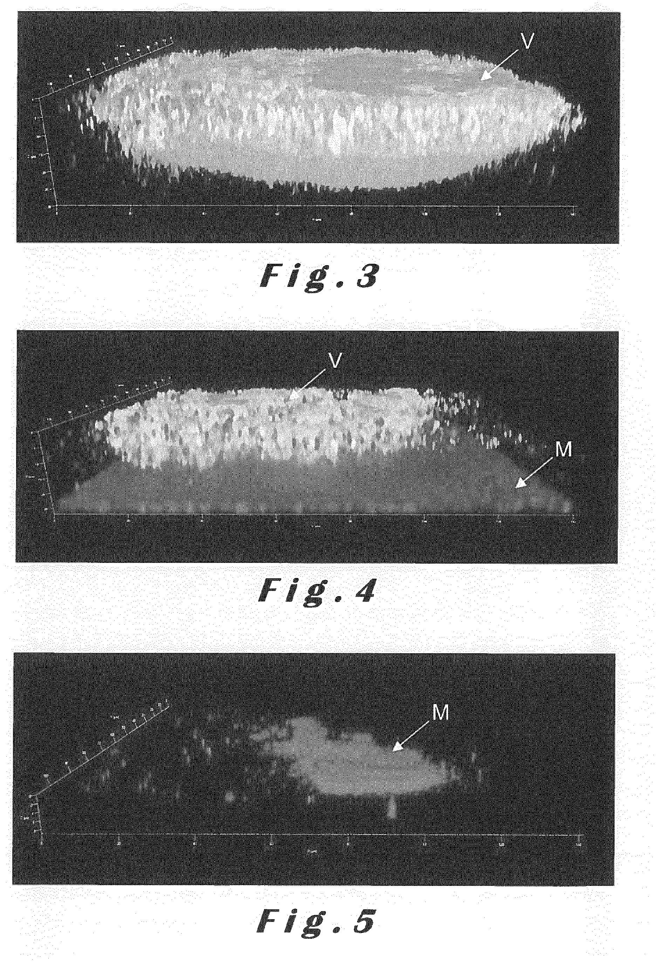

La figure 3 est une image de microscopie confocale pour un isolat de S. aureus (isolé au départ d'un pacemaker implanté) ayant développé un biofilm durant 24h.
La figure 4 est une image de microscopie confocale pour un isolat de S. aureus (isolé au départ d'un pacemaker implanté) ayant développé un biofilm durant 24h et ensuite traité avec une composition comprenant uniquement la ciprofloxacine comme molécule antibiotique à 1 mg/L durant 24h.
La figure 5 est une image de microscopie confocale pour un isolat de S. aureus (isolé au départ d'un pacemaker implanté) ayant développé un biofilm durant 24h et ensuite traité avec une composition selon l'invention (la ciprofloxacine à 1 mg/L + 0,025% de DNase I + 0,01% de dispersine B + 0,05% de cellulase) durant 24h.

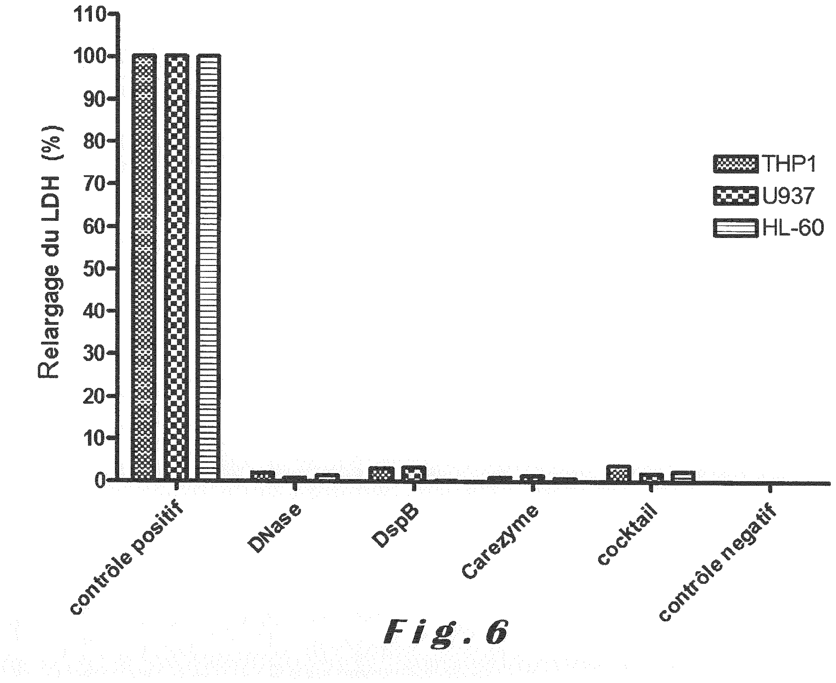

La figure 6 est un graphique qui illustre la cytotoxicité de la DNase I, de la dispersine B et de la cellulase seule ou associées sur des lignées cellulaires humaines.

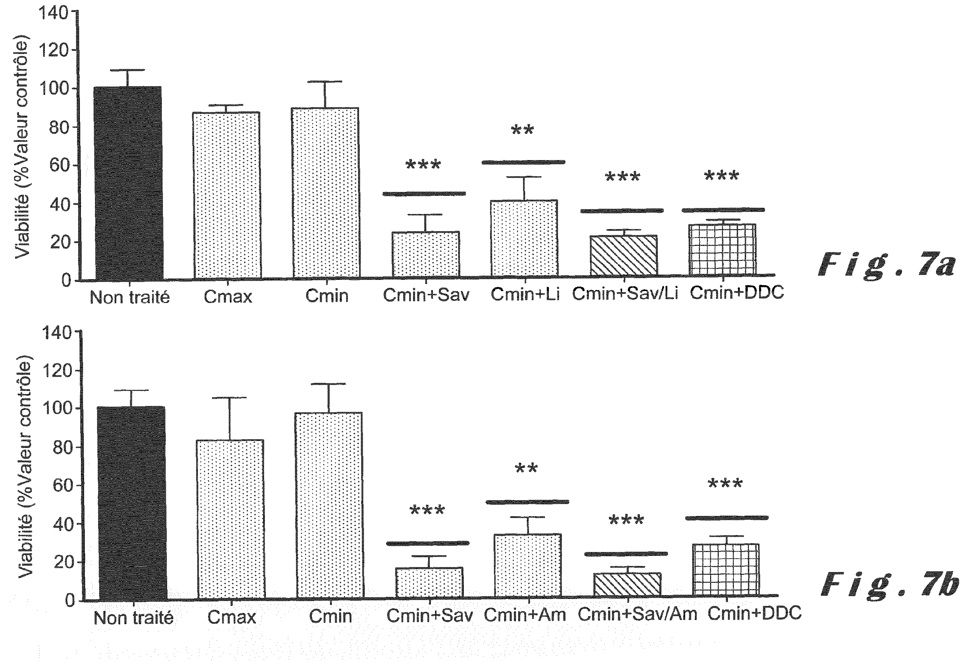

Les figures 7a à 7d sont des graphiques qui illustrent la viabilité (% de viabilité par rapport au contrôle) des bactéries d'un isolat de P. aeruginosa PA20 (isolé au départ d'un cathéter artériel implanté) ayant développé un biofilm, suite à une mise en contact avec différents antibiotiques à concentration Cmin en combinaison avec différentes enzymes ou cocktail d'enzymes.

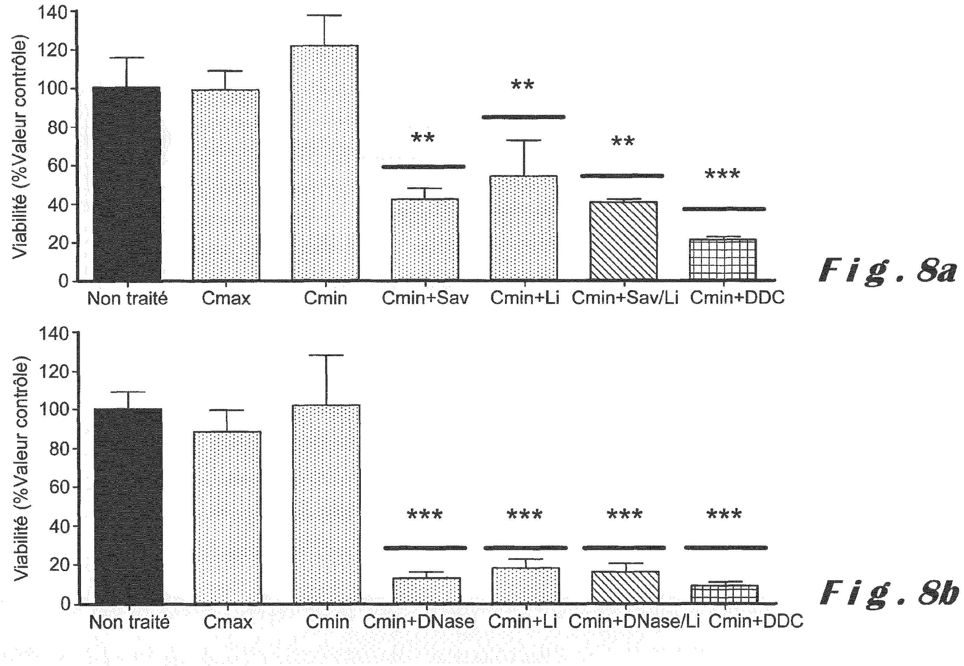

Les figures 8a à 8d sont des graphiques qui illustrent la viabilité (% de viabilité par rapport au contrôle) des bactéries d'un isolat de P. aeruginosa PA500 (isolé au départ d'un cathéter artériel implanté) ayant développé un biofilm, suite à une mise en contact avec différents antibiotiques à concentration Cmin en combinaison avec différentes enzymes ou cocktail d'enzymes.

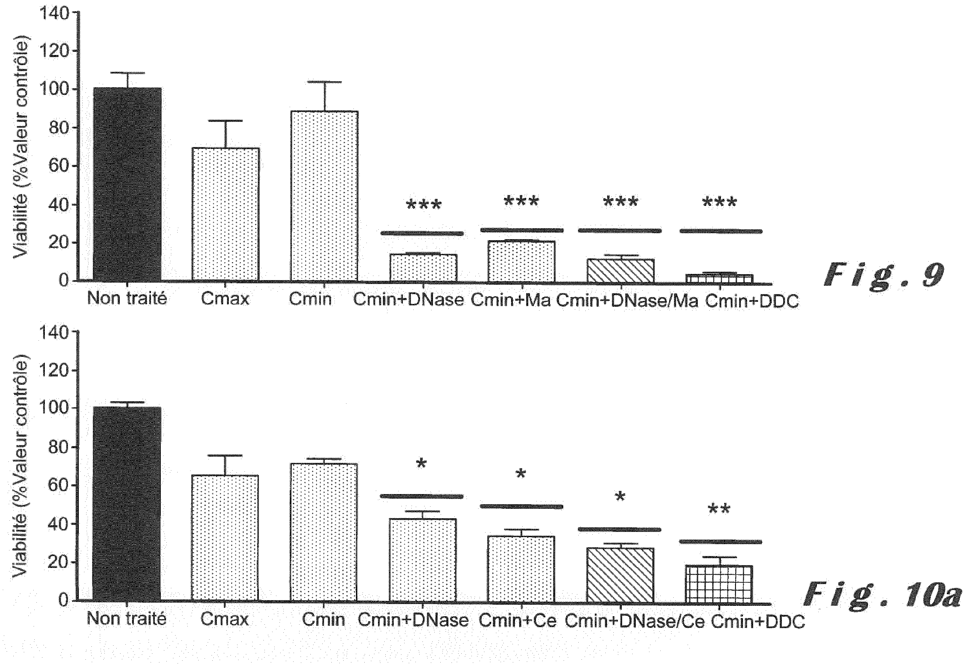

La figure 9 est un graphique qui illustre la viabilité (% de viabilité par rapport au contrôle) des bactéries d'un isolat de S. aureus Sa2003/1083 (isolé au départ d'une prothèse de genou) ayant développé un biofilm, suite à une mise en contact avec la ciprofloxacine comme antibiotique à concentration Cmin en combinaison avec différentes enzymes ou cocktail d'enzymes.

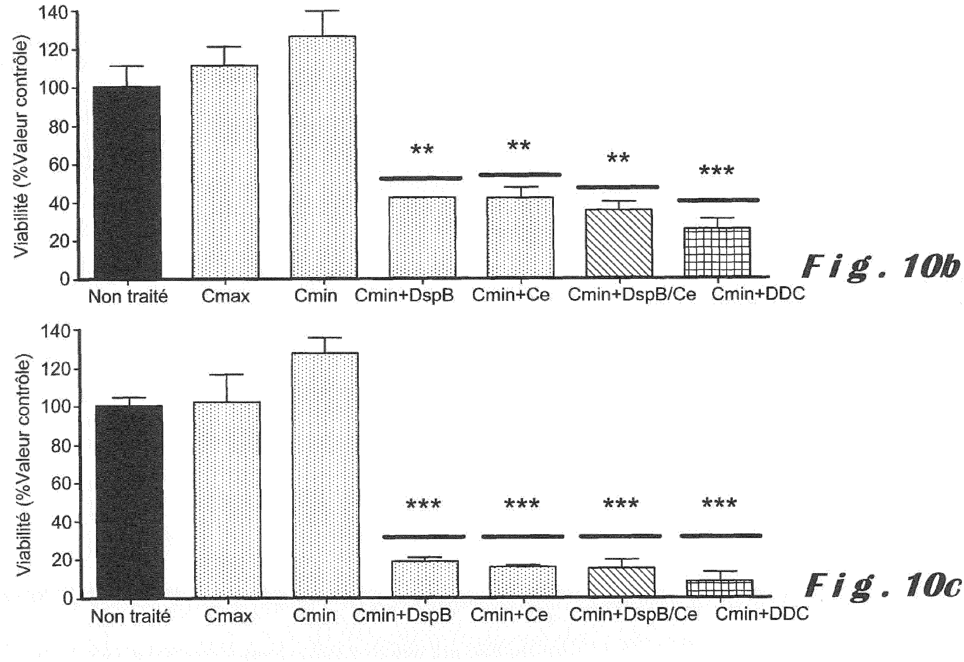

Les figures 10a à 10c sont des graphiques qui illustrent la viabilité (% de viabilité par rapport au contrôle) des bactéries d'un isolat de K. pneumoniae Kp826 (isolé au départ d'un cathéter veineux central) ayant développé un biofilm, suite à une mise en contact avec différents antibiotiques à concentration Cmin en combinaison avec différentes enzymes ou cocktail d'enzymes.

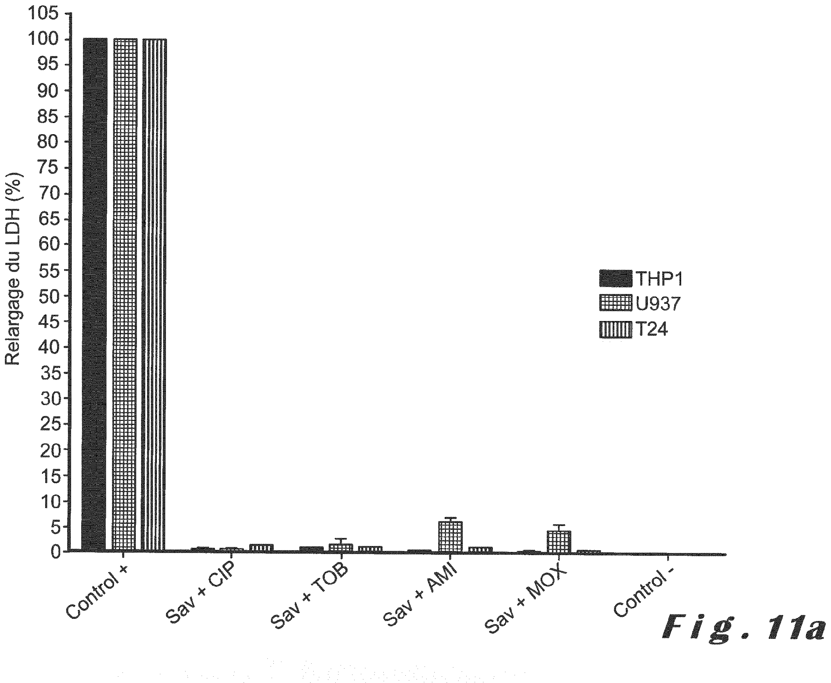

Les figures 11a à 11f sont des graphiques qui illustrent la cytotoxicité de compositions selon l'invention sur des lignées cellulaires humaines.

### Exemples

### EXEMPLE 1 : efficacité et cytotoxicité d'une première composition selon l'invention utilisée dans le traitement des infections post-implantatoires

### A. Efficacité

Afin de tester l'efficacité d'une composition selon l'invention comprenant au moins une enzyme et au moins une molécule biocide et/ou antibiotique (molécule microbicide) pour traiter les infections post-implantatoires du corps de mammifères, en particulier du corps humain, plusieurs expérimentations ont été menées au départ d'isolats prélevés sur des dispositifs médicaux infectés par des biofilms dans le corps humain suite à leur implantation. Ces isolats sont repris dans le tableau 1 ci-dessous.

Trois expérimentations différentes, réalisées selon trois modèles de biofilms ont été menées : (1) modèle statique de biofilm in-vitro, (2) modèle dynamique de biofilm in-vitro et (3) modèle dynamique de biofilm en bioréacteur.

Par ailleurs, des images de microscopie confocale ont été prises pour un isolat afin de visualiser la dispersion des bactéries et l'efficacité d'une composition selon l'invention en termes de survie des bactéries.

Pour réaliser ces expérimentations, différentes compositions selon l'invention ont été préparées sous agitation (120 RPM) par dilution d'au moins une molécule antibiotique et/ou biocide dans une solution aqueuse (eau tamponnée avec du trishydroxyméthylaminométhane 20 mM et présentant un pH de 7,5) comprenant au moins une enzyme. Pour les essais présentés ci-dessous, la ciprofloxacine a été utilisée comme molécule antibiotique.

Afin de juger de l'efficacité d'un traitement des infections post-implantatoires réalisé avec une composition selon l'invention, la viabilité des bactéries a été quantifiée en suivant l'évolution de la couleur de la résazurine (7-Hydroxy-3*H*-phenoxazin-3-one 10-oxide), cette couleur variant selon le potentiel rédox d'une solution qui dépend lui-même de l'activité microbienne. La quantification a été réalisée classiquement par photométrie d'absorption (mesure à 590 nm avec l'appareillage Spectramax M4). Sur base des valeurs mesurées pour différentes concentrations de la molécule antibiotique et/ou biocide en présence d'une concentration enzymatique fixée dans la composition selon l'invention, ont été déterminées les courbes doses-réponses et plus particulièrement le ECso, c'est-à-dire la concentration nécessaire en la molécule antibiotique et/ou biocide pour assurer une réduction de 50% de la viabilité des bactéries, ce qui correspond à 50% de réduction de la grandeur du signal mesuré par photométrie d'absorption.

**Tableau 1**

| **Isolat** | **Souche** | **Bactérie** | **Origine** |
|---|---|---|---|
| 1 | 80124430375 | *S.aureus MRSA* | pacemaker |
| 2 | 80224422456 | *S.aureus MRSA* | prothèse du genou |
| 3 | 80124474762 | *S.aureus MRSA* | vis du genou |
| 4 | 80224420266 | *S. epidermidis* | cathéter veineux central (CVC) |
| 5 | 6081 | *E.coli* | cathéter urinaire |
| 6 | 5701 | *E.coli* | implant orthopédique |
| 7 | 9794 | *E. faecalis* | cathéter urinaire |
| 8 | 9781 | *E. faecalis* | cathéter urinaire |
| 9 | 9555 | *E. faecalis* | cathéter urinaire |
| 10 | DIV5508 | *P.aeroginosa* | cathéter veineux central (CVC) |
| 11 | 04/190 | *P.aeroginosa* | cathéter artériel |

### 1. Modèle statique de biofilm in-vitro

Au départ de chacun des isolats mentionnés au tableau 1, des biofilms (n = 4) ont été développés durant 24 heures à une température de 37°C dans les puits d'une plaque 96-puits contenant 200 µL d'un milieu de culture TSB (Tryptic Soy Broth VWR) supplémenté avec 1% de glucose et 2% de chlorure de sodium.

Ensuite, durant une seconde période de 24 heures, les biofilms développés dans les puits ont été soumis à des concentrations croissantes (de 0,15 à 40 mg/L) de ciprofloxacine comme antibiotique dans une solution aqueuse comprenant 0,025% de DNase I (VWR), 0,01% de dispersine B (Symbiose Biomaterials) et 0,05% de cellulase (Carezyme® de Novozyme).

Précisons ici que les pourcentages en enzymes sont des pourcentages massiques (M/M) qui expriment les quantités en poids de chacune des enzymes commerciales par rapport au poids total de la composition. Ceci s'applique à l'ensemble du présent document.

Avant de procéder à la quantification de la viabilité des cellules bactériennes avec la résazurine, les plaques comportant les biofilms développés ont été lavées avec un tampon PBS (pH = 7,4) puis incubées en présence de résazurine (0,01 mg/L) durant 1 heure dans le noir.

Les résultats obtenus sont présentés dans le tableau 2 ci-dessous qui reprend, sur base des courbes doses-réponses établies, les concentrations (mg/L) nécessaires en la molécule antibiotique afin de réduire de 50% la viabilité des bactéries étudiées ayant développés un biofilm, aussi appelée EC₅₀ (ce qui correspond à 50% de réduction de la grandeur du signal mesuré par photométrie d'absorption).

L'augmentation de l'activité de la molécule antibiotique, soit la diminution de son ECso, lorsqu'elle est associée à 0,025% de DNase I, 0,01% de dispersine B et 0,05% de cellulase est également mentionnée au tableau 2 (rapport des EC₅₀ : solution B / solution A).

Comme on peut le constater, pour chacun des isolats testé, une composition selon l'invention permet systématiquement d'augmenter de façon significative l'activité de la molécule antibiotique, autrement dit de réduire la ECso de manière significative. Ces résultats indiquent, en comparaison avec une composition contenant uniquement la molécule antibiotique, que les bactéries sont atteintes et tuées par la molécule de façon bien plus efficace. Ceci s'explique par la présence des enzymes qui déstructurent véritablement le biofilm de telle façon à libérer les bactéries qui sont alors directement en contact avec la molécule antibiotique.

**Tableau 2**

| Isolat | Concentration en la molécule antibiotique dans une solution comprenant l'antibiotique seul (solution A) pour réduire de 50% la viabilité des bactéries (ECso) | Concentration en la molécule antibiotique dans une solution comprenant l'antibiotique en association avec 0,025% de DNase I, 0,01% de dispersine B et 0,05% de cellulase (solution B) pour réduire de 50% la viabilité des bactéries (EC₅₀) | Augmentation de l'activité de la molécule antibiotique |
|---|---|---|---|
| 1 | >40^{a} | 1,5 | >26,6 |
| 2 | 0,8 | 0,1 | 8 |
| 3 | 1,2 | 0,2 | 6 |
| 4 | 1,77 | 0,08 | 22,1 |
| 5 | 2,7 | 0,11 | 24,5 |
| 6 | >40 | 0,23 | >173,9 |
| 7 | >40 | 0,25 | >160 |
| 8 | >40 | 1,4 | >28,5 |
| 9 | >40 | 2,5 | >16 |
| 10 | >40 | 1,26 | >31 |
| 11 | >40 | 0,05 | >792 |

| | | | |
|---|---|---|---|
| ^{a}> 40 signifie qu'il n'y a pas eu de réduction de 50 % de la population microbienne pour une concentration en antibiotique de 40 mg/L. La EC₅₀ est donc supérieure à 40 mg/L | | | |

### 2. Modèle dynamique de biofilm in-vitro

20 µL de culture liquide des isolats 1 et 5 (n = 4) ont été inoculés dans les puits d'une plaque 96-puits contenant 180 µL d'un milieu de culture TSB (Tryptic Soy Broth VWR) supplémenté avec 1% de glucose et 2% de chlorure de sodium puis une plateforme PEG y a ensuite été plongée afin que des biofilms se développent sur les protubérances PEG durant 48 heures à une température de 37°C sous agitation constante à 120 RPM.

Ensuite, durant une seconde période de 24 heures, les biofilms développés dans les puits sur les protubérances ont été soumis, toujours sous agitation constante à 120 RPM, à des concentrations croissantes (de 0,15 à 40 mg/L) de ciprofloxacine comme antibiotique présent dans une solution aqueuse comprenant 0,025% de DNase I (VWR), 0,01% de dispersine B (Symbiose Biomaterials) et 0,05% de cellulase (Carezyme® de Novozyme).

Avant de procéder à la quantification de la viabilité des cellules bactériennes avec la résazurine, les plaques PEG avec les protubérances comportant les biofilms développés ont été incubées en présence de résazurine durant 2 heures dans le noir.

Les résultats obtenus sont présentés dans le tableau 3 ci-dessous qui reprend, sur base des courbes doses-réponses établies, les concentrations (mg/L) nécessaires en molécule antibiotique afin de réduire de 50% la viabilité des bactéries étudiées ayant développé un biofilm (ce qui correspond à 50% de réduction de la grandeur du signal mesuré par photométrie d'absorption). L'augmentation de l'activité de la molécule antibiotique lorsqu'elle est associée à 0,025% de DNase I, 0,01% de dispersine B et 0,05% de cellulase est également mentionnée au tableau 2 (rapport des concentrations EC₅₀ : solution B / solution A).

Comme on peut le constater, pour les isolats n°1 (S. aureus isolé au départ d'un pacemaker au niveau duquel s'est développé un biofilm dans le corps humain après implantation) et 5 (E. coli isolé au départ d'un cathéter urinaire au niveau duquel s'est développé un biofilm dans le corps humain après implantation), une composition selon l'invention permet systématiquement d'augmenter l'activité de la molécule antibiotique, soit de réduire la EC₅₀, de façon significative.

**Tableau 3**

| Isolat | Concentration en la molécule antibiotique dans une solution comprenant l'antibiotique seul (solution A) pour réduire de 50% la viabilité des bactéries (ECso) | Concentration en la molécule antibiotique dans une solution comprenant l'antibiotique en association avec 0,025% de DNase I, 0,01% de dispersine B et 0,05% de cellulase (solution B) pour réduire de 50% la viabilité des bactéries (EC₅₀) | Augmentation de l'activité de la molécule antibiotique |
|---|---|---|---|
| 1 | 0,7 | 0,001 | 700 |
| 5 | 3,18 | 0,004 | 795 |

Ces résultats indiquent, en comparaison avec une composition contenant uniquement la molécule antibiotique, que les bactéries sont atteintes et tuées par la molécule de façon bien plus efficace. Ceci s'explique par la présence des enzymes qui déstructurent véritablement le biofilm, de telle façon à libérer les bactéries qui sont alors directement en contact avec la molécule antibiotique.

### 3. Modèle dynamique de biofilm en bioréacteur

Les isolats 1 et 5 (n = 3) ont été inoculés sur des coupons en polycarbonate placés ensuite dans un bioréacteur CDC (BioSurfaces Technologies) contenant 300 ml d'un milieu de culture TSB (Tryptic Soy Broth VWR) supplémenté avec 1% de glucose et 2% chlorure de sodium afin que s'y développent des biofilms durant 20 heures à une température de 37°C sous agitation à 120 RPM. Le développement a plus particulièrement été opéré en deux phases successives, à savoir (1) une première phase d'incubation de 6 heures à une concentration bactérienne de 10⁵ bactéries/ml et (2) une deuxième phase de 14 heures durant laquelle a été réalisée une circulation continue à raison de 10 ml/minute du milieu de culture dans le bioréacteur à l'aide d'une pompe péristaltique (Masterflex). Suite à ce développement des biofilms réalisé en deux phases, ces derniers ont été soumis, dans le bioréacteur, à une concentration de 3,2 mg/L de ciprofloxacine comme antibiotique présent dans une solution aqueuse comprenant 0,025% de DNase I (VWR), 0,01% de dispersine B (Symbiose Biomaterials) et 0,05% de cellulase (Carezyme® de Novozyme).

Les coupons ont ensuite été retirés de façon aseptique après 0, 4, 8, 12, 18 et 24 heures et rincés deux fois dans un tampon PBS avant sonication des biofilms formés. Des dilutions successives dans un tampon PBS (pH = 7,4) ont alors été réalisées au départ des échantillons ainsi obtenus avant étalement sur un milieu de culture TSA (VWR) en vue d'un comptage des colonies bactériennes (Log₁₀ CFU/ml) suite à une incubation des milieux de culture durant 18 heures à une température de 37°C et à une humidité relative de 60%.

Les résultats obtenus sont présentés aux figures 1 et 2 d'où il ressort à nouveau clairement que, pour chacun des isolats testé, une composition selon l'invention permet systématiquement d'augmenter de façon significative l'activité de la molécule antibiotique. En effet, en ce qui concerne l'isolat n°1 (S. aureus isolé au départ d'un pacemaker au niveau duquel s'est développé un biofilm dans le corps humain après implantation), une réduction logarithmique du nombre de bactéries vivantes de l'ordre de 5 a été observée en comparaison avec une réduction logarithmique de l'ordre de 0,5 lorsque la molécule antibiotique est utilisée seule (voir figure 1). Une même observation a été effectuée pour l'isolat n°5 (E. coli isolé au départ d'un cathéter urinaire au niveau duquel s'est développé un biofilm dans le corps humain après implantation) : réduction logarithmique du nombre de bactéries vivantes de l'ordre de 5 lors d'un traitement avec une composition selon l'invention et de l'ordre de 0,5 pour une composition comprenant l'antibiotique seul (voir figure 2).

Ces résultats indiquent, en comparaison avec une composition contenant uniquement la molécule antibiotique, que les bactéries sont atteintes et tuées par la molécule de façon bien plus efficace. Ceci s'explique par la présence des enzymes qui vont véritablement déstructurer le biofilm de telle façon à libérer les bactéries qui sont alors directement en contact avec la molécule antibiotique.

### 4. Images de microscopie confocale

Pour l'isolat n°1 (S. *aureus* isolé au départ d'un pacemaker au niveau duquel s'est développé un biofilm dans le corps humain après implantation), des images confocales ont été prises et correspondent aux figures 3 à 5. La figure 3 correspond à une prise de vue du biofilm développé par l'isolat n°1 pendant 24h; la figure 4 correspond à une prise de vue du biofilm développé pendant 24h par l'isolat n°1 après traitement (incubation) avec une composition contenant uniquement la molécule antibiotique (ciprofloxacine à 1 mg/L) durant 24h; la figure 5 correspond à une prise de vue du biofilm développé pendant 24h par l'isolat n°1 après traitement (incubation) avec une composition selon l'invention (ciprofloxacine à 1 mg/L + 0,025% de DNase I + 0,01% de dispersine B + 0,05% de cellulase) durant 24h.

Avant de réaliser les différentes prises de vue, une coloration a été réalisée avec le kit LIVE/DEAD (Invitrogen) durant 30 min avant rinçage avec un tampon PBS.

Il ressort de ces images confocales, comme pour les essais avec les modèles de biofilms ci-dessus, qu'une composition selon l'invention est nettement plus efficace qu'une composition ne comprenant que la molécule antibiotique. En effet, la figure 5 (traitement avec une composition suivant l'invention) montre que toutes les bactéries sont tuées (détruites) (M) alors que la figure 4 (traitement avec la molécule antibiotique seule) montre au contraire que de nombreuses bactéries sont encore vivantes (V). Comme précédemment, ceci s'explique par la présence des enzymes qui vont véritablement déstructurer le biofilm de telle façon à libérer les bactéries qui sont alors directement en contact avec la molécule antibiotique.

### B. Cytotoxicité

Afin de juger de la cytotoxicité d'une composition selon l'invention utilisée dans le traitement des infections post-implantatoires du corps de mammifères, en particulier du corps humain, trois lignées cellulaires humaines THP-1, U937 et HL-60 ont été utilisées. Pour chaque lignée, des cellules (10⁴ cellules/ml) ont été incubées durant 4h dans les puits d'une plaque 96-puits en présence d'un cocktail enzymatique selon l'invention (0,025% de DNase I + 0,01% de dispersine B + 0,05% de cellulase) ou en présence de chacune de ces enzymes à une même concentration que celle selon laquelle elles sont présentes dans le cocktail enzymatique (DNase I - DNase ou dispersine B - DispB ou cellulase - Carezyme).

La cytotoxicité a été évaluée sur base de la mesure de la lactate déshydrogénase (LDH) présente dans le surnageant en utilisant le kit de détection PLUS (Roche, Basel, Suisse). Le taux de LDH naturellement relargué par les cellules non traitées de chaque lignée a été mesuré (contrôle négatif) ainsi que le taux maximal de LDH libéré par ces mêmes cellules (contrôle positif). La cytotoxicité a été calculée selon la formule suivante : (valeur mesurée pour l'échantillon - valeur du contrôle négatif) / (valeur du contrôle positif - valeur du contrôle négatif) * 100.

Les résultats obtenus sont présentés à la figure 6 où on peut constater que le cocktail enzymatique tout comme chacune des enzymes selon l'invention ne présentent pas de toxicité pour les lignées cellulaires humaines.

### EXEMPLE 2 : efficacité et cytotoxicité de différentes compositions selon l'invention utilisées dans le traitement des infections post-implantatoires

D'autres compositions que celle décrite à l'exemple 1 ont été testées en termes d'efficacité et de cytotoxicité, ceci afin d'établir que différentes combinaisons d'enzymes avec différentes molécules microbicides (antibiotiques) permettent bien d'assurer le traitement des infections post-implantatoires. L'objectif des essais réalisés dans cet exemple 2 est essentiellement de mettre en avant que la présente invention ne se limite pas à une composition particulière telle que celle de l'exemple 1 mais que toute une série de combinaisons différentes et donc de compositions différentes selon l'invention sont efficaces dans le traitement des infections post-implantatoires.

Différentes souches cliniques ont été isolées au départ de dispositifs médicaux infectés par des biofilms dans le corps humain suite à leur implantation. Ces isolats ainsi que leur origine sont repris au tableau 4 ci-dessous.

**Tableau 4**

| **Souche** | **Espèce bactérienne** | **Origine** |
|---|---|---|
| PA20 | *P. aeruginosa* | cathéter artériel |
| PA500 | *P. aeruginosa* | cathéter artériel |
| Sa2003/1083 | *S. aureus MRSA* | prothèse de genou |
| Kp826 | *K. pneumoniae* | cathéter veineux central |

### A. Efficacité : modèle statique de biofilm in-vitro

### a) Mise en culture et formation de biofilm au départ des isolats de P. aeruginosa (PA20 et PA500)

5 µl d'un stock glycérolé de PA20 ou de PA500 ont été ajoutés à 5 ml de milieu de culture LB-glucose (milieu composé d'un mélange de Luria Bertani (LB) et de glucose 1%) et incubés pendant 24h à 37°C. La suspension bactérienne a ensuite été diluée avec le milieu LB-glucose afin d'obtenir un inoculum de 10⁶ bactéries/ml.

Les biofilms ont été cultivés sur des plaques à 96 puits suite à un ajout d'un volume de 200 µl de l'inoculum dans chaque puit. Les plaques ont alors été incubées à 37°C pendant 48h avec un renouvellement du milieu après 24h.

Les biofilms obtenus ont ensuite été traités pendant 24h avec différentes enzymes (dispersine B, DNase, cellulase (carezyme), savinase (protéase), lipolase (lipase), amylase (stainzyme) et mannanase) en combinaison avec différents antibiotiques à concentration maximale (Cmax) ou à concentration minimale (Cmin), Cmax étant la concentration sérique maximale après l'administration de l'antibiotique et Cmin la concentration sérique minimale. Ces concentrations Cmin et Cmax sont basées sur celles recommandées par l'EUCAST (The European Committee on Antimicrobial Susceptibility Testing).

Les traitements du biofilm à l'aide de différentes combinaisons d'un antibiotique avec une ou plusieurs enzymes ont été réalisés en respectant les concentrations suivantes en antibiotiques et en enzymes :
- enzymes : 0,05% ; DCC = 0,025% DNase + 0,01% Dispersine B (DspB) + 0,05% cellulase (Ce)
- amikacine : Cmin = 5 mg/L (Cmax = 24 mg/L)
- tobramycine : Cmin = 0,9 mg/L (Cmax = 4 mg/L)
- moxifloxacine : Cmin = 0,3 mg/L (Cmax = 3,6 mg/L)
- méropénème : Cmin = 0,1 mg/L (Cmax = 20 mg/L)
- ciprofloxacine : Cmin = 0,6 mg/L (Cmax = 3,2 mg/L)

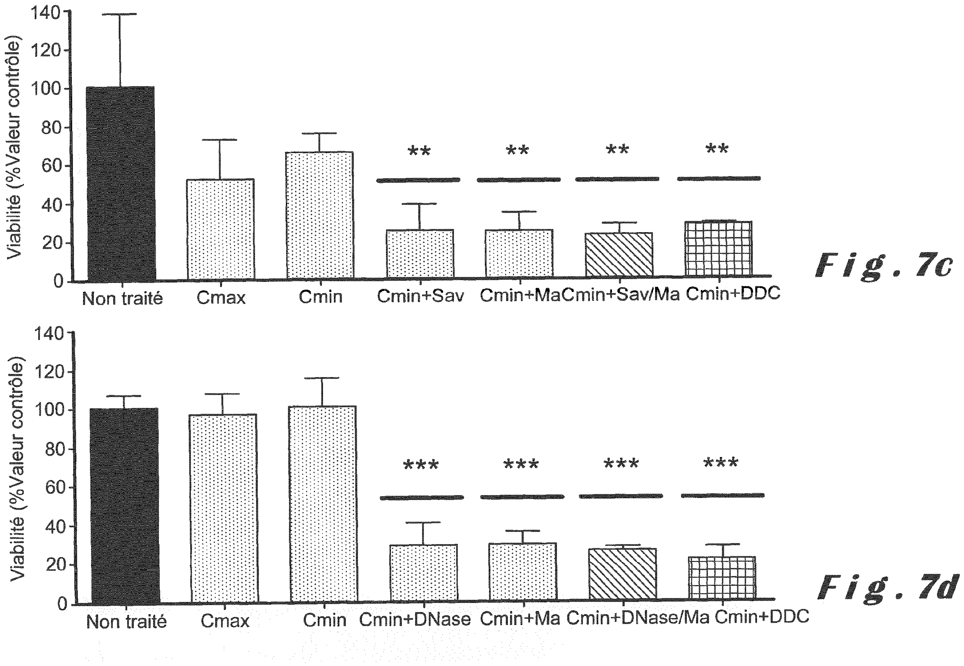

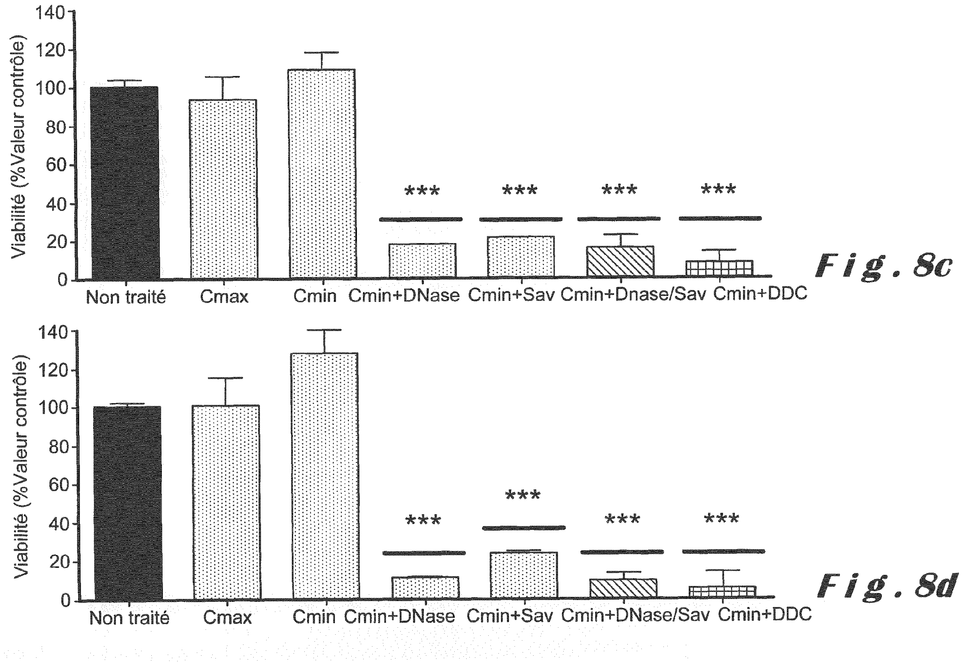

La fluorescéine diacétate (FDA) a été utilisée pour quantifier les bactéries viables de P. aeruginosa (PA20 ou PA500). Les plaques comportant les biofilms ont été lavées deux fois par un tampon MOPS puis incubées en présence de FDA (100 µg/ml) durant 1h à l'abri de la lumière. La FDA est un composé non fluorescent hydrolysable en un composant jaune fluorescent (la fluorescéine) par des estérases intracellulaires non spécifiques produites par les bactéries viables. La quantification a été réalisée par spectrophotométrie (longueur d'onde d'excitation à 494 nm et émission à 518 nm) avec un spectramax M4. La quantité de fluorescéine mesurée par fluorimétrie est directement proportionnelle au nombre de bactéries viables dans le milieu. Les résultats obtenus sont présentés aux figures 7 et 8.

Les figures 7a à 7d illustrent la viabilité (% de viabilité par rapport au contrôle - non traité) des bactéries d'un isolat de P. aeruginosa PA20 (isolé au départ d'un cathéter artériel implanté) ayant développé un biofilm, suite à une mise en contact avec
- (A) de l'amikacine à concentration Cmin comme antibiotique en combinaison avec de la savinase (Sav) comme seule enzyme ou avec de la lipolase (Li) comme seule enzyme ou avec de la savinase (Sav) et de la lipolase (Li) comme enzymes ou avec de la Dispersine B (DspB), de la DNase et de la cellulase (Ce) comme enzymes (DCC);
- (B) de la tobramycine à concentration Cmin comme antibiotique en combinaison avec de la savinase (Sav) comme seule enzyme ou avec de l'amylase (Am) comme seule enzyme ou avec de la savinase (Sav) et de l'amylase (Am) comme enzymes ou avec de la Dispersine B (DspB), de la DNase et de la cellulase (Ce) comme enzymes (DCC) ;
- (C) de la moxifloxacine à concentration Cmin comme antibiotique en combinaison avec de la savinase (Sav) comme seule enzyme ou avec de la mannanase (Ma) comme seule enzyme ou avec de la savinase (Sav) et de la mannanase (Ma) comme enzymes ou avec de la Dispersine B (DspB), de la DNase et de la cellulase (Ce) comme enzymes (DCC) ;
- (D) du méropénème à concentration Cmin comme antibiotique en combinaison avec de la DNase comme seule enzyme ou avec de la mannanase (Ma) comme seule enzyme ou avec de la DNase et de la mannanase (Ma) comme enzymes ou avec de la Dispersine B (DspB), de la DNase et de la cellulase (Ce) comme enzymes (DCC).

Les figures 8a à 8d illustrent la viabilité (% de viabilité par rapport au contrôle - non traité) des bactéries d'un isolat de P. aeruginosa PA500 (isolé au départ d'un cathéter artériel implanté) ayant développé un biofilm, suite à une mise en contact avec
- (A) de la ciprofloxacine à concentration Cmin comme antibiotique en combinaison avec de la savinase (Sav) comme seule enzyme ou avec de la lipolase (Li) comme seule enzyme ou avec de la savinase (Sav) et de la lipolase (Li) comme enzymes ou avec de la Dispersine B (DspB), de la DNase et de la cellulase (Ce) comme enzymes (DCC) ;
- (B) de l'amikacine à concentration Cmin comme antibiotique en combinaison avec de la DNase comme seule enzyme ou avec de la lipolase (Li) comme seule enszyme ou avec de la DNase et de la lipolase (Li) comme enzymes ou avec de la Dispersine B (DspB), de la DNase et de la cellulase (Ce) comme enzymes (DCC) ;
- (C) de la tobramycine à concentration Cmin comme antibiotique en combinaison avec de la DNase comme seule enzyme ou avec de la savinase (Sav) comme seule enzyme ou avec de la DNase et de la savinase (Sav) comme enzymes ou avec de la Dispersine B (DspB), de la DNase et de la cellulase (Ce) comme enzymes (DCC) ;
- (D) de la moxifloxacine à concentration Cmin comme antibiotique en combinaison avec de la DNase comme seule enzyme ou avec de la savinase (Sav) comme seule enzyme ou avec de la DNase et de la savinase (Sav) comme enzymes ou avec de la Dispersine B (DspB), de la DNase et de la cellulase (Ce) comme enzymes (DCC).

Sur les graphiques des figures 7a à 7d et 8a à 8d, sont représentés les résultats d'un test statistique (One-way ANOVA avec comparaison multiple - test de Dunnett) mis en œuvre afin de déterminer si une différence significative est observée entre la Cmax de l'antibiotique et la Cmin de l'antibiotique associé avec une ou plusieurs enzymes. Les caractères *, ** et *** signifient repectivement p<0,05, p<0,01 et p<0,001.

De ces graphiques, il peut être observé que toutes les combinaisons [antibiotique à Cmin + enzyme(s)] testées permettent de réduire au moins de façon significative (valeur p au moins inférieur à 0,05) la viabilité des bactéries d'espèce P. aeruginosa (ceci tant pour la souche PA20 que pour la souche PA500 de P. aeruginosa) en comparaison avec l'antibiotique seul à une concentration Cmax. De même, il peut être constaté qu'une différence significative est observée entre toutes les combinaisons [antibiotique (Cmin) + enzyme(s)] testées et le contrôle (biofilm non traité), ce qui n'est pas le cas pour une comparaison entre l'effet de l'antibiotique seul (à Cmin ou Cmax) et le contrôle (biofilm non traité).

Ceci montre l'intérêt de combiner un antibiotique et au moins une enzyme pour le traitement préventif et/ou curatif d'infections post-implantatoires à un site d'implantation, lesdites infections étant des infections post-implantatoires.

### b) Mise en culture et formation de biofilm au départ des isolats de S. aureus (Sa2003/1083) et K. pneumoniae (Kp826)

5 µl d'un stock glycérolé de Sa2003/1083 ou de Kp826 ont été ajoutés à 5 ml de milieu TGN (milieu composé d'un mélange de Tryptic Soy Broth, de glucose 1% et de NaCl 2%) et incubés pendant 24h à 37°C. La suspension bactérienne est diluée avec le milieu TGN afin d'obtenir un inoculum de 10⁶ bactéries/ml.

Les biofilms ont été cultivés sur des plaques à 96 puits suite à un ajout d'un volume de 200 µl de l'inoculum dans chaque puit. Les plaques ont été incubées à 37°C pendant 24h.

Les biofilms obtenus ont ensuite été traités pendant 24h avec les différentes enzymes en combinaison avec différents antibiotiques à la Cmax ou à la Cmin.

Les traitements du biofilm à l'aide de différentes combinaisons d'un antibiotique avec une ou plusieurs enzymes ont été réalisés en respectant les concentrations suivantes en antibiotiques et en enzymes :
- enzymes : 0,05% ; DCC = 0,025% DNase + 0,01% Dispersine B (DspB) + 0,05% cellulase (Ce)
- amikacine : Cmin = 5 mg/L (Cmax = 24 mg/L)
- tobramycine : Cmin = 0,9 mg/L (Cmax = 4 mg/L)
- moxifloxacine : Cmin = 0,3 mg/L (Cmax = 3,6 mg/L)
- méropénème : Cmin = 0,1 mg/L (Cmax = 20 mg/L)
- ciprofloxacine : Cmin = 0,6 mg/L (Cmax = 3,2 mg/L)

Les plaques comportant les biofilms ont été lavées deux fois par un tampon PBS puis incubées durant 30 minutes à l'abri de la lumière en présence de 200 µl de résazurine (10 µg/ml) dans chaque puit. La résazurine (7-Hydroxy-3H-phenoxazin-3-one 10-oxide) est un colorant non toxique de couleur bleue qui peut diffuser dans les bactéries et ensuite être réduit en résorufine, un composé fluorescent. La viabilité des bactéries a donc été quantifiée en suivant l'évolution de la fluorescence de la résorufine qui est directement proportionnelle au nombre de bactéries viables dans le milieu.

La quantification a été réalisée classiquement par spectrophotométrie (longueur d'onde d'excitation à 560 nm et émission à 590 nm) avec spectramax M4. Les résultats obtenus sont présentés aux figures 9 et 10a à 10c.

La figure 9 illustre la viabilité (% de viabilité par rapport au contrôle - non traité) des bactéries d'un isolat de S. aureus Sa2003/1083 (isolé au départ d'une prothèse de genou) ayant développé un biofilm, suite à une mise en contact avec de la ciprofloxacine à concentration Cmin comme antibiotique en combinaison avec de la DNase comme seule enzyme ou avec de la mannanase (Ma) comme seule enzyme ou avec de la DNase et de la mannanase (Ma) comme enzymes ou avec de la Dispersine B (DspB), de la DNase et de la cellulase (Ce) comme enzymes (DCC).

Les figures 10a à 10c illustrent la viabilité (% de viabilité par rapport au contrôle) des bactéries d'un isolat de K. pneumoniae Kp826 (isolé au départ d'un cathéter veineux central) ayant développé un biofilm, suite à une mise en contact avec
- (A) de la ciprofloxacine à concentration Cmin comme antibiotique en combinaison avec de la DNase comme seule enzyme ou avec de la cellulase (Ce) comme seule enzyme ou avec de la DNase et de la cellulase (Ce) comme enzymes ou avec de la Dispersine B (DspB), de la DNase et de la cellulase (Ce) comme enzymes (DCC) ;
- (B) de l'amikacine à concentration Cmin comme antibiotique en combinaison avec de la Dispersine B (DspB) comme seule enzyme ou avec de la cellulase (Ce) comme seule enzyme ou avec de Dispersine B (ispB) et de la cellulase (Ce) comme enzymes ou avec de la Dispersine B (DspB), de la DNase et de la cellulase (Ce) comme enzymes (DCC) ;
- (C) de la tobramycine à concentration Cmin comme antibiotique en combinaison avec de la Dispersine B (DspB) comme seule enzyme ou avec de la cellulase (Ce) comme seule enzyme ou avec de Dispersine B (DspB) et de la cellulase (Ce) comme enzymes ou avec de la Dispersine B (DspB), de la DNase et de la cellulase (Ce) comme enzymes (DCC).

Sur les graphiques des figures 9a à 9c, sont représentés les résultats d'un test statistique (One-way ANOVA avec comparaison multiple - test de Dunnett) mis en œuvre afin de déterminer si une différence significative est observée entre la Cmax de l'antibiotique et la Cmin de l'antibiotique associé avec une ou plusieurs enzymes. Les caractères *, ** et *** signifient repectivement p<0,05, p<0,01 et p<0,001.

De ces graphiques, il peut être observé que toutes les combinaisons [antibiotique à Cmin + enzyme(s)] testées permettent de réduire au moins de façon significative (valeur p au moins inférieur à 0,05) la viabilité des bactéries d'espèce S. aureus Sa2003/1083 et d'espèce K. pneumoniae Kp862 en comparaison avec l'antibiotique seul à une concentration Cmax. De même, il peut être constaté qu'une différence significative est observée entre toutes les combinaisons [antibiotique (Cmin) + enzyme(s)] testées et le contrôle (biofilm non traité), ce qui n'est pas le cas pour une comparaison entre l'effet de l'antibiotique seul (à Cmin ou Cmax) et le contrôle (biofilm non traité).

Ceci montre à nouveau, pour deux autres espèces bactériennes l'intérêt de combiner un antibiotique et au moins une enzyme pour le traitement préventif et/ou curatif d'infections post-implantatoires à un site d'implantation, lesdites infections étant des infections post-implantatoires.

### B. Cytotoxicité

Un protocole identique à celui décrit sous le point B. de l'exemple 1 a été mis en œuvre afin de juger de la cytotoxicité de différentes combinaisons d'un antibiotique et d'une enzyme sur des lignées cellulaires humaines THP1, U937 et T24. Les résultats obtenus sont présentés aux figures 11a à 11f. Sur ces graphiques, le contrôle négatif permet de déterminer l'activité de la LDH libérée par des cellules normales non traitées (LDH de libération spontannée) et le contrôle positif permet de déterminer l'activité maximale de la LDH libérée par des cellules traitées avec une solution de lyse cellulaire. Pour réaliser ces essais, les concantrations suivantes ont été respectées :
- enzymes: 0,05% ; DCC = 0,025% DNase + 0,01% Dispersine B (DspB) + 0,05% cellulase (Ce)
- amikacine : C min = 5 mg/L
- tobramycine : C min = 0,9 mg/L
- moxifloxacine : C min = 0,3 mg/L
- méropénème : C min = 0,1 mg/L
- ciprofloxacine : C min = 0,6 mg/L

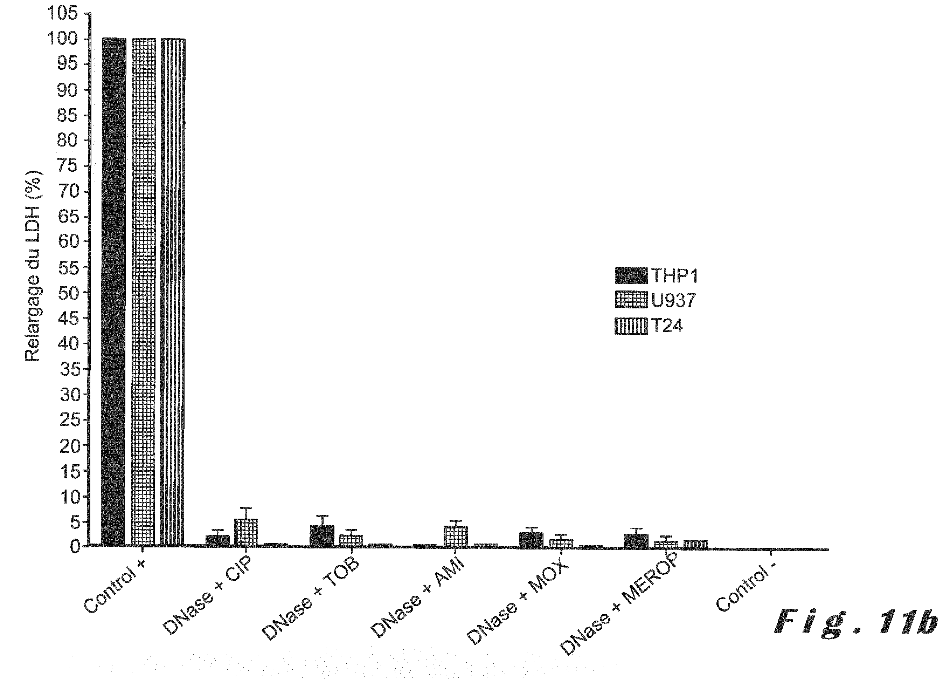

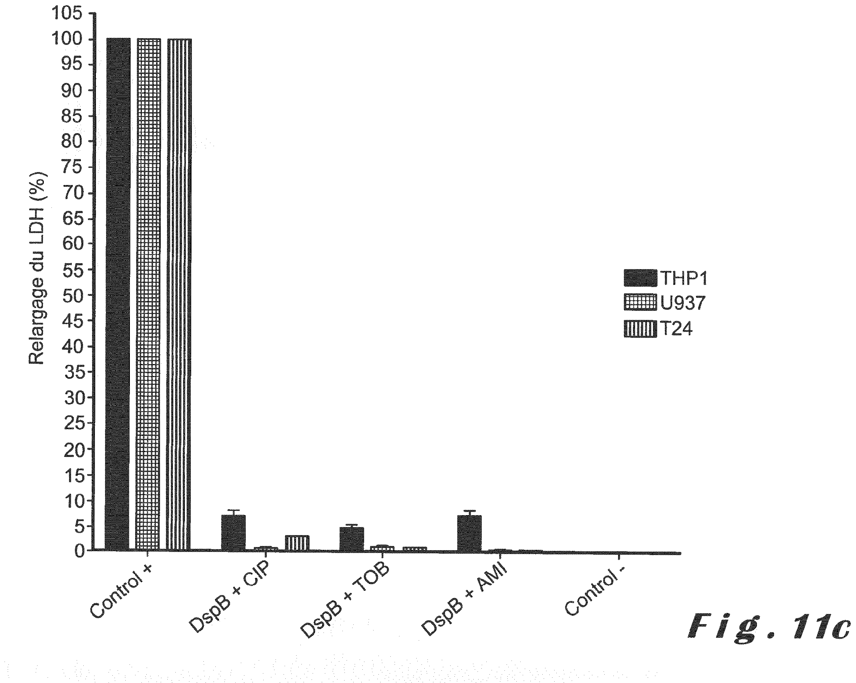

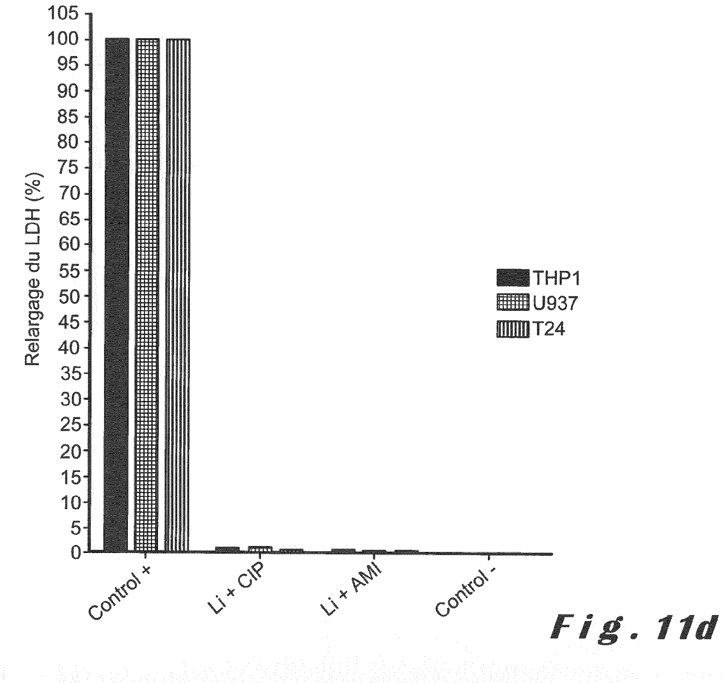

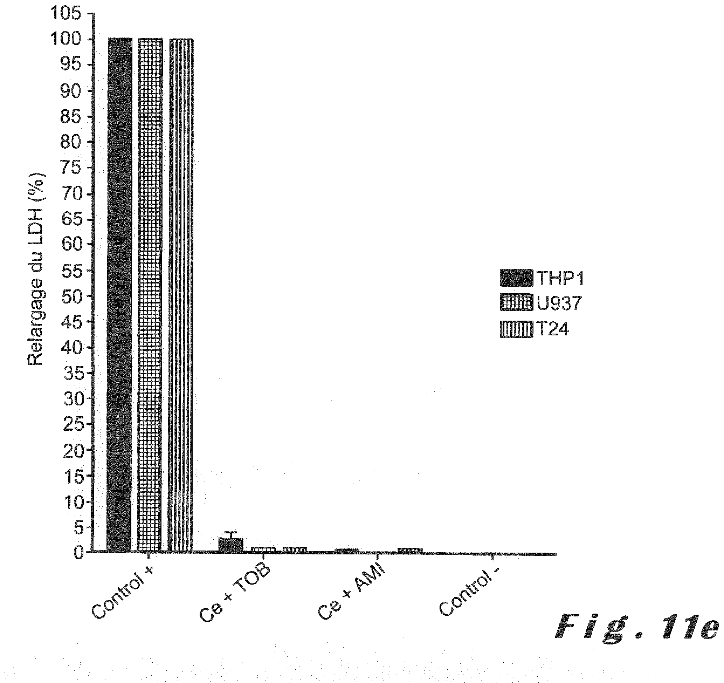

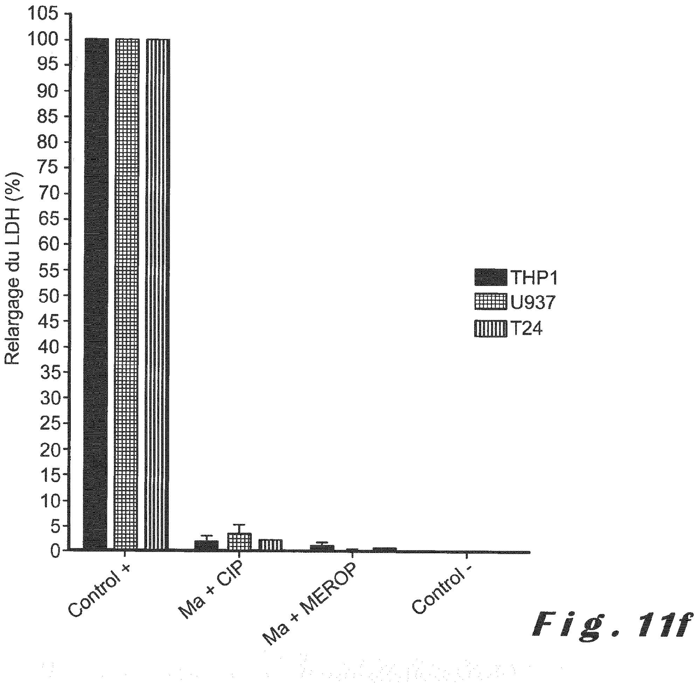

La figure 11a illustre le relargage du LDH (%) des lignées cellulaires humaines THP1, U937 et T24 pour la savinase (Sav) en combinaison avec l'antibiotique ciprofloxacine (CIP) ou l'antibiotique tobramycine (TOB) ou l'antibiotique amikacine (AMI) ou l'antibiotique moxifloxacine (MOX). La figure 11b illustre le relargage du LDH (%) des lignées cellulaires humaines THP1, U937 et T24 pour la DNase en combinaison avec l'antibiotique ciprofloxacine (CIP) ou l'antibiotique tobramycine (TOB) ou l'antibiotique amikacine (AMI) ou l'antibiotique moxifloxacine (MOX) ou l'antibiotique méropénème (MEROP). La figure 11c illustre le relargage du LDH (%) des lignées cellulaires humaines THP1, U937 et T24 pour la Dispersine B (DspB) en combinaison avec l'antibiotique ciprofloxacine (CIP) ou l'antibiotique tobramycine (TOB) ou l'antibiotique amikacine (AMI). La figure 11d illustre le relargage du LDH (%) des lignées cellulaires humaines THP1, U937 et T24 pour la lipolase (Li) en combinaison avec l'antibiotique ciprofloxacine (CIP) ou l'antibiotique amikacine (AMI). La figure 11e illustre le relargage du LDH (%) des lignées cellulaires humaines THP1, U937 et T24 pour la cellulase (Ce) en combinaison avec l'antibiotique ciprofloxacine tobramycine (TOB) ou l'antibiotique amikacine (AMI). La figure 11f illustre le relargage du LDH (%) des lignées cellulaires humaines THP1, U937 et T24 pour la mannanase (Ma) en combinaison avec l'antibiotique ciprofloxacine (CIP) ou l'antibiotique méropénème (MEROP).

De ces graphiques des figures 11a à 11f, il peut être conclu qu'aucune des combinaison [antibiotique + enzyme] ne présente de toxicité pour les lignées cellulaires humaines testées.

### C. Conclusion de l'exemple 2

De l'exemple 2, il ressort très clairement que la présente invention ne se limite pas à une composition particulière telle que celle de l'exemple 1 mais que toute une série de combinaisons différentes et donc de compositions différentes selon l'invention sont efficaces dans le traitement des infections post-implantatoires. Par ailleurs, il a été démontré que les compositions selon l'invention ne sont pas cytotoxiques.

Il est bien entendu que la présente invention n'est en aucune façon limitée aux formes de réalisations décrites ci-dessus et que bien des modifications peuvent y être apportées sans sortir du cadre des revendications annexées.

## Revendications

1. Composition comprenant au moins une enzyme et au moins une molécule microbicide choisie dans le groupe constitué des fluoroquinolones, des glucopeptides, des lipoglucopeptides, de l'acide fusidique, des pénicillines, des céphalosporines, des carbapenèmes, des monobactames, des polymixines, des béta-lactames, des macrolides, des lincosamides, des oxazolidinones, des phénicoles, des tétracyclines, des aminoglycosides, des rifamycines, des nitrofuranes, des sulfamides, des nitroimidazoles, et de leurs mélanges comme produits de combinaison, pour un emploi simultané, séparé ou échelonné dans le temps, pour utilisation dans le traitement curatif d'infections dans les tissus, l'os et les cartilages et/ou les muqueuses corporelles internes à un site d'implantation, lesdites infections étant des infections post-implantatoires du corps de mammifères, en particulier des infections post-implantatoires du corps humain, ladite au moins une enzyme étant une désoxyribonucléase (DNase).

2. Composition pour utilisation selon la revendication 1, dans laquelle ladite au moins une enzyme est présente en une concentration comprise entre 0,01 et 1000 mg/L.

3. Composition pour utilisation selon la revendication 1 ou 2, dans laquelle ladite au moins une molécule microbicide antibiotique est présente en une concentration comprise entre 0,01 et 1000 mg/L.

4. Composition pour utilisation selon l'une quelconque des revendications 1 à 3, ladite composition se présentant sous forme d'une solution stérile aqueuse injectable ou non, à diluer ou non dans de l'eau pour préparation injectable ou sous forme d'une poudre soluble, préférentiellement sous forme d'un lyophilisat.

5. Composition pour utilisation selon l'une quelconque des revendications 1 à 3, ladite composition se présentant sous forme de gel.

6. Composition pour utilisation selon l'une quelconque des revendications 1 à 5, lesdites infections post-implantatoires du corps de mammifères, en particulier du corps humain, étant des infections post-implantatoires de bactéries Staphylococcus aureus, Staphylococcus epidermis, Escherichia coli, Enterococcus faecalis, Klebsiella pneumoniae et Pseudomonas aeruginosa.

7. Composition pour utilisation selon l'une quelconque des revendications 1 à 6, lesdites infections post-implantatoires du corps de mammifères, en particulier du corps humain, étant des infections localisées à un site d'implantation d'un dispositif médical implantable, par exemple d'une sonde, d'un cathéter ou d'une prothèse.

8. Composition pour utilisation selon l'une quelconque des revendications 1 à 7, prévue pour abattre d'au moins 1 Log10, de préférence d'au moins 2 Log10, préférentiellement d'au moins 5 Log10 des microorganismes responsables d'infections post-implantatoires du corps de mammifères, en particulier du corps humain.

9. Composition pour utilisation selon revendication 1, dans laquelle une administration est effectuée par injection, par infiltration, par irrigation, par ingestion ou par application percutanée.

## Patentansprüche

1. Zusammensetzung, mindestens ein Enzym und mindestens ein mikrobizides Molekül umfassend, das aus der Gruppe bestehend aus den Fluorchinolonen, Glucopeptiden, Lipoglucopeptiden, Fusidinsäure, Penizillinen, Cephalosporinen, Carbapenemen, Monobactamen, Polymixinen, Beta-Lactamen, Macroliden, Lincosamiden, Oxazolidinonen, Phenicolen, Tetracyclinen, Aminoglycosiden, Rifamycinen, Nitrofuranen, Sulfamiden, Nitroimidazolen, und deren Gemischen als Kombinationsprodukte ausgewählt wird, für einen gleichzeitigen, getrennten oder zeitlich gestaffelten Einsatz, für eine Verwendung bei der Heilbehandlung von Infektionen in den Geweben, den Knochen, und den Knorpeln und/ oder inneren Körperschleimhäuten an einer Implantationsstelle, wobei die Infektionen Postimplantationsinfektionen des Körpers von Säugern, insbesondere Postimplantationsinfektionen des menschlichen Körpers sind, wobei das mindestens eine Enzym eine Desoxyribonuklease (DNase) ist.

2. Zusammensetzung zur Verwendung nach Anspruch 1, wobei das mindestens eine Enzym in einer Konzentration vorhanden ist, die zwischen 0,01 und 1000 mg/L enthalten ist.

3. Zusammensetzung zur Verwendung nach Anspruch 1 oder 2, wobei das mindestens eine mikrobizide antibiotische Molekül in einer Konzentration vorhanden ist, die zwischen 0,01 und 1000 mg/L enthalten ist.

4. Zusammensetzung zur Verwendung nach einem der Ansprüche 1 bis 3, wobei sich die Zusammensetzung in Form einer injizierbaren oder nicht injizierbaren, im Wasser zu verdünnenden oder nicht zu verdünnenden sterilen wässrigen Lösung zur injizierbaren Herstellung oder in Form eines löslichen Pulvers, vorzugsweise in Form eines Lyophilisats darstellt.

5. Zusammensetzung zur Verwendung nach einem der Ansprüche 1 bis 3, wobei sich die Zusammensetzung in Form eines Gels darstellt.

6. Zusammensetzung zur Verwendung nach einem der Ansprüche 1 bis 5, wobei die Postimplantationsinfektionen des Körpers von Säugern, insbesondere des menschlichen Körpers, die Postimplantationsinfektionen von Staphylococcus aureus-, Staphylococcus epidermis-, Escherichia coli, Enterococcus faecalis, Klebsiella pneumoniae und Pseudomonas aeruginosa Bakterien sind.

7. Zusammensetzung zur Verwendung nach einem der Ansprüche 1 bis 6, wobei die Postimplantationsinfektionen des Körpers von Säugern, insbesondere des menschlichen Körpers, Infektionen sind, die an einer Implantationsstelle einer implantierbaren medizinischen Vorrichtung, beispielsweise einer Sonde, eines Katheters oder eine Prothese gelegen sind.

8. Zusammensetzung zur Verwendung nach einem der Ansprüche 1 bis 7, vorgesehen zur Beseitigung mindestens 1 Log10, vorzugsweise mindestens 2 Log10, vorzugsweise mindestens 5 Log10 der Mikroorganismen, die für Postimplantationsinfektionen des Körpers von Säugern, insbesondere des menschlichen Körpers verantwortlich sind.

9. Zusammensetzung zur Verwendung nach Anspruch 1, wobei eine Verabreichung durch Injektion, durch Infiltration, durch Irrigation, durch Schlucken, oder durch perkutane Verabreichung durchgeführt wird.

## Claims

1. Composition comprising at least one enzyme and at least one microbicidal molecule selected from the group consisting of the fluoroquinolones, glycopeptides, lipoglycopeptides, fusidic acid, penicillins, cephalosporins, carbapenems, monobactams, polymyxins, beta-lactams, macrolides, lincosamides, oxazolidinones, phenicols, tetracyclines, aminoglycosides, rifamycins, nitrofurans, sulphamides, nitroimidazoles, and mixtures thereof as combination products, for simultaneous use, separate use or use staggered over time, for use in the therapeutic treatment of infections in the tissues, bones and cartilages and/or the internal bodily mucosae at an implantation site, said infections being post-implantation infections of the body of mammals, in particular post-implantation infections of the human body, said at least one enzyme being a deoxyribonuclease (DNase).

2. Composition for use according to claim 1, in which said at least one enzyme is present at a concentration between 0.01 and 1000 mg/L.

3. Composition for use according to claim 1 or 2, in which said at least one antibiotic microbicidal molecule is present at a concentration between 0.01 and 1000 mg/L.

4. Composition for use according to any one of claims 1 to 3, said composition being in the form of a sterile aqueous solution, injectable or not, to be diluted or not in water for an injectable preparation or in the form of a soluble powder, preferably in the form of a lyophilizate.

5. Composition for use according to any one of claims 1 to 3, said composition being in the form of gel.

6. Composition for use according to any one of claims 1 to 5, said post-implantation infections of the body of mammals, in particular of the human body, being post-implantation infections with bacteria Staphylococcus aureus, Staphylococcus epidermis, Escherichia coli, Enterococcus faecalis, Klebsiella pneumoniae and Pseudomonas aeruginosa.

7. Composition for use according to any one of claims 1 to 6, said post-implantation infections of the body of mammals, in particular of the human body, being infections localized to an implantation site of an implantable medical device, for example a cannula, a catheter or a prosthesis.

8. Composition for use according to any one of claims 1 to 7, provided for killing at least 1 Log10, preferably at least 2 Log10, preferably at least 5 Log10 of the microorganisms responsible for post-implantation infections of the body of mammals, in particular of the human body.

9. Composition for use according to claim 1, in which administration is carried out by injection, by infiltration, by irrigation, by ingestion or by percutaneous application.
